(19) Europäisches Patentamt
European Patent Office
Office européen des brevets

(11) Veröffentlichungsnummer : **0 347 381 B1**

# (12) EUROPÄISCHE PATENTSCHRIFT

(45) Veröffentlichungstag der Patentschrift :
**12.02.92 Patentblatt 92/07**

(21) Anmeldenummer : **89810430.2**

(22) Anmeldetag : **07.06.89**

(51) Int. Cl.$^5$ : **C07D 317/30,** C07D 319/06, C07C 69/66, C08F 20/18, G03F 7/004

## (54) Ungesättigte beta-Ketoesteracetale und ihre Anwendungen.

(30) Priorität : **13.06.88 CH 2257/88**

(43) Veröffentlichungstag der Anmeldung :
**20.12.89 Patentblatt 89/51**

(45) Bekanntmachung des Hinweises auf die Patenterteilung :
**12.02.92 Patentblatt 92/07**

(84) Benannte Vertragsstaaten :
**BE CH DE FR GB IT LI NL SE**

(56) Entgegenhaltungen :
**EP-A- 0 022 571**
**EP-A- 0 031 566**

(56) Entgegenhaltungen :
**EP-A- 0 202 196**
**DE-A- 2 342 068**
**DE-A- 2 718 254**
**CHEMICAL ABSTRACTS, Band 87, Nr 9, 29 August 1977, Seite 551,Zusammenfassung Nr 68333n, Columbus, Ohio, USA.**

(73) Patentinhaber : **CIBA-GEIGY AG**
**Klybeckstrasse 141**
**CH-4002 Basel (CH)**

(72) Erfinder : **Schulthess, Adrian, Dr.**
**Uf em Bärg**
**CH-1734 Tentlingen (CH)**
Erfinder : **Hunziker, Max, Dr.**
**Chasseralstrasse 8**
**CH-3186 Düdingen (CH)**

EP 0 347 381 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ungesättigte β-Ketoesteracetale, die daraus durch Homo- oder Copolymerisation erhaltenen polymeren β-Ketoesteracetale, positive Photoresists enthaltend die polymeren β-Ketoesteracetale als Lösungsinhibitoren und Verwendung der Photoresists zur Herstellung von gedruckten Schaltungen, integrierten Schaltkreisen, Druckformen oder als silberlose photographische Filme.

Bei den wässrig entwickelbaren Photoresists wird bekanntlich ein filmbildendes, wässrig oder wässrigalkalisch lösliches Harz mit einem Lösungsinhibitor gemischt, der die Löslichkeit des Gemisches in einem wässrig-alkalischen Entwickler stark herabsetzt. Durch eine photochemische Reaktion verändert sich der Lösungsinhibitor derart, zum Beispiel durch Depolymerisation, Acetalspaltung, Orthoesterspaltung oder Bildung einer Carbonsäure aus einem o-Chinondiazid, dass der Photoresist an den belichteten Stellen in dem Entwickler wieder gut löslich ist.

Bekannte Lösungsinhibitoren für positive Photoresists sind beispielsweise Naphthochinondiazide (vgl. J. Kosar in "Light-Sensitive Systems", Verlag John Wiley & Sons, New York 1965, Seite 339 bis 352) in Novolakharzen. Naphthochinone sind photoaktiv und wirken an unbelichteten Stellen im Photoresist als Lösungsinhibitor. Doch sind sie temperaturempfindlich und weisen nur eine geringe Lagerstabilität auf. Da sie ausserdem Licht sehr stark absorbieren, können sie nicht in dicken Schichten angewendet werden.

Im US-Patent 3 779 778 werden für positive Photoresists Lösungsinhibitoren beschrieben, die durch Anlagerung eines Bis- oder Polyphenols, einer Monoalkylsulfonamidgruppen enthaltenden Verbindung oder von bestimmten sekundären Aminen an Vinylethergruppen enthaltenden Verbindungen, insbesondere an Tetrahydropyranylether, erhalten werden. Abgesehen davon, dass die Synthese dieser Lösungsinhibitoren aufwendig ist, sind diese in dicken Schichten relativ unempfindlich und benötigen lange Belichtungszeiten.

In der DE-AS 27 18 254 werden als Lösungsinibitoren für positive Photoresists Polyadditions- oder Polykondensationsprodukte mit wiederkehrenden Acetal- oder Ketaleinheiten in der Hauptkette offenbart. Auch diese Lösungsinhibitoren sind, wenn der Photoresist nicht in dünnen Schichten angewandt wird, nicht mehr sehr empfindlich, so dass relativ lange Belichtungszeiten in Kauf genommen werden müssen.

Die aus der DE-OS 28 29 511 bekannten Lösungsinhibitoren sind niedermolekulare Enoletherverbindungen. Monomere Lösungsinhibitoren bergen die Gefahr in sich, dass sie aus dem Trägerpolymer herausdiffundieren, so dass photographische Filme, die solche Lösungsinhibitoren enthalten, nicht sehr haltbar sind.

Auch die in der EP-A 0 202 196 offenbarten cyclischen Acetale und Ketale von β-Ketoestern sind monomere Verbindungen und somit mit dem gleichen Nachteil behaftet wie die zuvor genannten Lösungsinhibitoren für Photoresists.

Es wurde gefunden, dass bestimmte ungesättigte β-Ketoesteracetale allein oder zusammen mit anderen olefinisch ungesättigten Verbindungen zu polymeren β-Ketoesteracetalen polymerisiert werden können, die wertvolle Lösungsinhibitoren für positiv arbeitende Photoresistsysteme darstellen.

Gegenstand vorliegender Erfindung sind somit neue Verbindungen der Formel I oder II

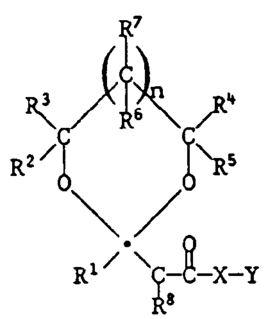

(I)

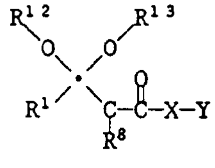

(II),

worin n für Null oder die Zahl 1 oder 2 steht,

$R^1$ ein Wasserstoffatom, ein unsubstituiertes oder durch Halogenatome substituiertes $C_1$-$C_{10}$-Alkyl, ein durch Ethersauerstoffatome unterbrochenes $C_2$-$C_{10}$-Alkyl oder ein unsubstituiertes oder durch Halogenatome, Nitro-

gruppen, $C_1$-$C_4$-Alkyl oder $C_1$-$C_4$-Alkoxy substituiertes Phenyl oder Benzyl bedeutet,
$R^2$, $R^3$, $R^4$, $R^5$, $R^6$, $R^7$ und $R^8$ unabhängig voneinander je ein Wasserstoffatom, Halogenatom, unsubstituiertes oder durch Halogenatome substituiertes $C_1$-$C_{10}$-Alkyl, unsubstituiertes oder durch Halogenatome, Cyano-, Nitrogruppen oder $C_1$-$C_4$-Alkyl substituiertes Phenyl oder Naphthyl oder einen Rest der Formeln -COOR$^9$,

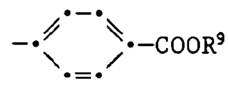

oder -SO$_2$R$^9$ bedeuten, worin
$R^9$ ein unsubstituiertes oder durch Halogenatome oder Nitrogruppen substituiertes $C_1$-$C_6$-Alkyl oder Phenyl bedeutet,
X für O, S oder NR$^{10}$ steht, wobei $R^{10}$ ein Wasserstoffatom, ein unsubstituiertes oder durch Halogenatome oder Nitrogruppen substituiertes $C_1$-$C_6$-Alkyl oder Phenyl bedeutet, und
Y für einen Rest der folgenden Formeln

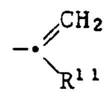 , 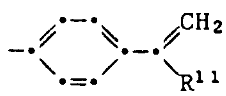 , 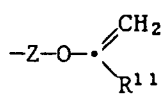 , 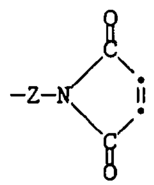 , 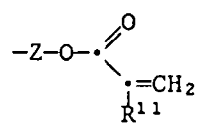 ,

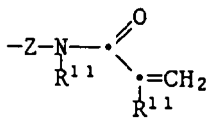 , 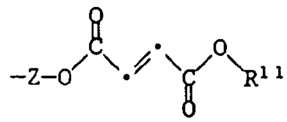 oder 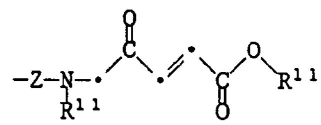

steht, worin $R^{11}$ ein Wasserstoffatom, ein unsubstituiertes oder durch Halogenatome oder Nitrogruppen substituiertes $C_1$-$C_6$-Alkyl oder Phenyl bedeutet,
Z für einen unsubstituierten oder durch Halogenatome, Nitrogruppen oder Phenyl substituierten, mindestens zwei Methylengruppen enthaltenden aliphatischen Rest oder einen unsubstituierten oder durch Halogenatome oder Nitrogruppen substituierten, mindestens zwei Methylengruppen enthaltenden durch Ethersauerstoffatome, Phenylen oder Cyclohexylengruppen unterbrochenen aliphatischen Rest steht,
$R^{12}$ und $R^{13}$ unabhängig voneinander je ein unsubstituiertes oder durch Halogenatome oder Nitrogruppen substituiertes $C_1$-$C_{10}$-Alkyl, Phenyl oder Naphthyl bedeuten.

Bevorzugte Verbindungen der Formel I oder II sind solche, worin n für Null oder die Zahl 1 steht, $R^1$ ein Wasserstoffatom, ein unsubstituiertes oder durch Chloratom(e) substituiertes $C_1$-$C_4$-Alkyl, ein durch Ethersauerstoffatom(e) unterbrochenes $C_2$-$C_4$-Alkyl, Phenyl oder Benzyl bedeutet, $R^2$, $R^3$, $R^4$, $R^5$, $R^6$ und $R^7$ unabhängig voneinander je ein Wasserstoffatom, Chloratom, $C_1$-$C_6$-Alkyl oder Phenyl bedeuten, $R^8$ ein Wasserstoffatom, Phenyl, p-Nitrophenyl, p-Cyanophenyl oder den Rest -COOR$^9$ bedeutet, worin $R^9$ für ein $C_1$-$C_6$-Alkyl oder Phenyl steht, X für ein Sauerstoffatom steht, Y für einen Rest der Formeln

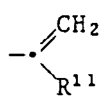 , 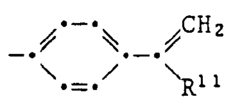

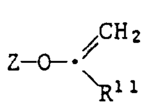 oder 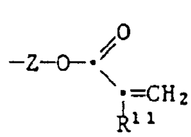

steht, worin $R^{11}$ ein Wasserstoffatom oder ein $C_1$-$C_4$-Alkyl bedeutet, Z für einen der folgenden Reste

$$-(CH_2)_b- ,$$

$-CH_2-CH_2-(OCH_2-CH_2)_c-OCH_2-CH_2-$ , $-CH_2-CHR^{11}-$ , 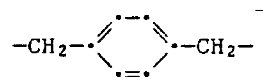

oder

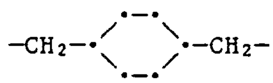

steht, worin b eine Zahl von 3 bis 5 und c Null oder eine Zahl von 1 bis 3 bedeuten, $R^{12}$ und $R^{13}$ unabhängig voneinander je ein $C_1$-$C_4$-Alkyl bedeuten.

Verbindungen der Formel I, worin n für Null oder die Zahl 1 steht, $R^1$ ein $C_1$-$C_4$-Alkyl, Methoxymethyl oder Phenyl bedeutet, $R^2$, $R^3$, $R^4$, $R^5$, $R^6$, $R^7$ und $R^8$ je ein Wasserstoffatom bedeuten, X für ein Sauerstoffatom steht und Y den Rest

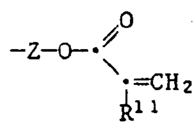

bedeutet, worin Z für Ethylen und $R^{11}$ für ein Wasserstoffatom oder Methyl stehen, stellen besonders bevorzugte Verbindungen dar.

Desgleichen sind von den Verbindungen der Formel II solche besonders bevorzugt, worin $R^1$, $R^{12}$ und $R^{13}$ unabhängig voneinander je ein $C_1$-$C_4$-Alkyl bedeuten, $R^8$ für ein Wasserstoffatom steht, X für ein Sauerstoffatom steht und Y einen Rest der Formel

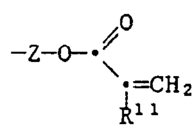

bedeutet, worin Z für Ethylen und $R^{11}$ für ein Wasserstoffatom oder Methyl steht.

Bedeuten $R^1$ bis $R^{11}$ ein unsubstituiertes oder substituiertes Alkyl, so handelt es sich um geradkettige oder um verzweigtkettige Reste, wie beispielsweise Methyl, Ethyl, 2-Chlor- oder 2-Bromethyl, n-Propyl, Isopropyl, n-Butyl, sek.-Butyl, tert.-Butyl, n-Pentyl, Isoamyl, n-Hexyl, n-Octyl oder 2-Nitroethyl.

Beispiele für die Reste $R^1$ bis $R^{11}$ als substituiertes Phenyl oder substituiertes Benzyl sind 2-, 3- oder 4-Chlorphenyl, 2-, 3- oder 4-Chlorbenzyl, 2-, 3- oder 4-Nitrophenyl, 2-, 3- oder 4-Cyanophenyl, 2-, 3- oder 4-Nitrobenzyl und 2- oder 4-Methoxybenzyl.

Stehen die Reste $R^2$ bis $R^8$ für ein substituiertes Naphthyl, so handelt es sich beispielsweise um $\alpha$-Chlor- oder $\alpha$-Nitronaphthyl.

Z als aliphatischer Rest steht beispielsweise für ein Alkylen, wie Ethylen, Isopropylen oder Butylen, oder für ein Oxyalkylen oder Poly(oxy)alkylen, die 2 bis 10 $C_2$- bis $C_5$-Oxyalkyleneinheiten aufweisen.

Die Herstellung der erfindungsgemässen, ungesättigten $\beta$-Ketoesteracetale erfolgt nach an sich bekannten Verfahren. Die erfindungsgemässen Verbindungen der Formeln I und II können beispielsweise und werden vorzugsweise hergestellt, indem man

a) eine Verbindung der Formel III

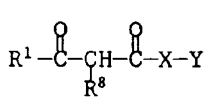 (III),

worin $R^1$, $R^8$, X und Y die gleiche Bedeutung wie in Formel I haben, entweder mit einer Verbindung der Formel IV

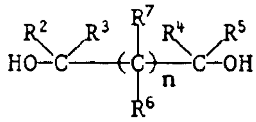

(IV),

worin n, $R^2$, $R^3$, $R^4$, $R^5$, $R^6$ und $R^7$ die gleiche Bedeutung wie in Formel I haben, zu einer Verbindung der Formel I umsetzt, wobei man auf 1 Mol der Verbindung der Formel III mindestens 1 Mol einer Verbindung der Formel IV einsetzt, oder mit Verbindungen der Formeln V und VI

$$HO\text{-}R^{12} \qquad (V)$$

und

$$OH\text{-}R^{13} \qquad (VI),$$

worin $R^{12}$ und $R^{13}$ die gleich Bedeutung wie in Formel II haben, zu einer Verbindung der Formel II umsetzt, wobei man auf 1 Mol der Verbindung III mindestens 2 Mol der Verbindungen der Formeln V und VI einsetzt,

b) Verbindungen der Formel VII oder VIII

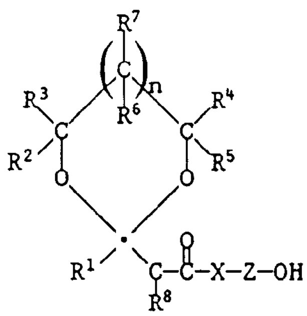

(VII)

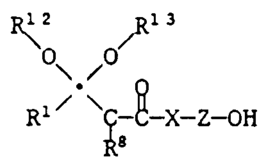

(VIII),

worin n, $R^1$, $R^2$, $R^3$, $R^4$, $R^5$, $R^6$, $R^7$, $R^8$, $R^{12}$, $R^{13}$, X und Z die gleiche Bedeutung wie in Formeln I und II haben, mit einer Verbindung der Formel IX, X oder XI

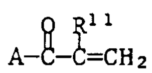

(IX)

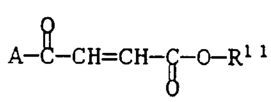

(X) oder

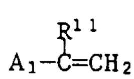

(XI),

worin $R^{11}$ die gleiche Bedeutung wie in Formel I oder II hat, A für ein Halogenatom oder eine Hydroxylgruppe steht, oder wobei die Verbindungen der Formeln IX und X auch als Anhydrid vorliegen können, und $A_1$ für ein Halogenatom oder ein $C_1$-$C_4$-Alkoxy steht, in molaren Mengen zu Verbindungen der Formel I oder II umsetzt, oder indem man

c) Verbindungen der Formel XII oder XIII

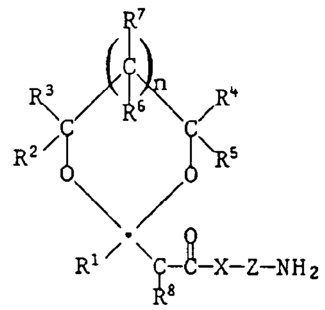

(XII)

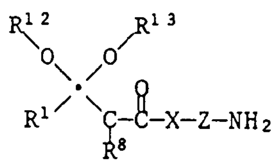

(XIII),

worin n, $R^1$, $R^2$, $R^3$, $R^4$, $R^5$, $R^6$, $R^7$, $R^8$, $R^{12}$, $R^{13}$, X und Z die gleiche Bedeutung wie in Formeln I und II haben, mit Maleinsäureanhydrid, einer Verbindung der Formel IX oder X oder deren Anhydrid in molaren Mengen zu Verbindungen der Formel I oder II umsetzt.

Die Acetalisierung einer Verbindung der Formel III mit einer Verbindung der Formel IV oder Verbindungen der Formeln V und VI erfolgt vorzugsweise in Gegenwart eines sauren Katalysators, beispielsweise einer Säure, wie Schwefelsäure, Salzsäure, Essigsäure oder p-Toluolsulfonsäure. Ausserdem können als Katalysatoren auch Salze der p-Toluolsulfonsäure, beispielsweise Pyridinium-p-toluolsulfonat (PPTS), Salze der Salzsäure oder Hydrochloride von Aminen verwendet werden. Die Umsetzungsreaktion kann in Ab- oder Anwesenheit eines Lösungsmittels durchgeführt werden. Für die Herstellung der ungesättigten β-Ketoesteracetale der Formel I verwendet man als Lösungsmittel vorzugsweise solche, welche Azeotrope mit Wasser bilden, wie beispielsweise Dichlormethan, Benzol, Toluol, Xylol oder p-Chlorbenzol. Für die Herstellung der ungesättigten β-Ketoacetale der Formel II verwendet man als Lösungsmittel vorzugsweise den zum Acetalisieren dienenden Alkohol.

Die Acetalisierung einer Verbindung der Formel III kann im Prinzip nach allen gängigen Verfahren durchgeführt werden. Beispielsweise durch Reaktion eines β-Ketoesters mit Orthoestern, wobei man je nach Reaktionsführung Acetale oder Enolether des betreffenden β-Ketoesters erhält, Umacetalisierung mit anderen Acetalen unter Säurekatalyse und Abdestillieren des entstehenden Ketons sowie durch Reaktion eines Enolethers von einem β-Ketoester unter Säure- oder Basenkatalyse mit einem Alkohol.

Die Verbindungen der Formeln III, IV, V und VI stellen bekannte Verbindungen dar, die zum Teil im Handel erhältlich sind oder nach bekannten Methoden hergestellt werden können. Eine Verbindung der Formel III lässt sich beispielsweise herstellen, indem man Acetessigsäureethylester mit Ethylenglykol umestert und den erhaltenen Acetessigsäure-β-hydroxyethylester mit Methacrylsäure oder einem aktivierten Säurederivat der Methacrylsäure verestert, oder indem man β-Hydroxyethylmethacrylat mit einem Diketen umsetzt.

Die Verbindungen der Formeln VII, VIII, XII und XIII sind ebenfalls bekannt und können beispielsweise hergestellt werden, indem man Acetale der Formel VIIa oder VIIIa

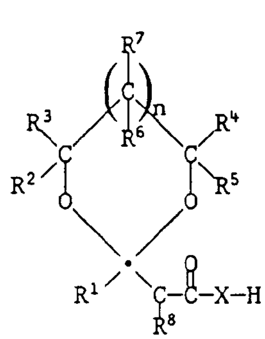

(VIIa)

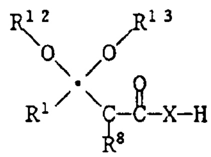

(VIIIa),

worin n, $R^1$, $R^2$, $R^3$, $R^4$, $R^5$, $R^6$, $R^7$, $R^8$, $R^{12}$, $R^{13}$ und X die gleiche Bedeutung wie in Formeln I und II haben, mit einem Diol der Formel HO-Z-OH, einem Aminoalkohol der Formel $HO\text{-}Z\text{-}NH_2$ oder einem Diamin der Formel $H_2N\text{-}Z\text{-}NH_2$, worin Z in den Formeln die gleiche Bedeutung wie in Formel VII oder VIII hat, in molaren Mengen umsetzt.

Die Verbindungen der Formeln IX bis XI stehen für (Meth)Acrylsäure und ihre Derivate (IX), Maleinsäure und ihre Derivate (X) sowie für Vinylverbindungen, wie beispielsweise Vinylether oder Vinylchlorid.

Wie eingangs erwähnt, lassen sich aus den Acetalen der Formeln I und II durch Polymerisation, gegebenenfalls in Gegenwart von olefinisch ungesättigten Comonomeren, polymere β-Ketoesteracetale herstellen.

Gegenstand vorliegender Erfindung sind somit auch polymere β-Ketoesteracetale mit einem Molekulargewicht (Mw) von 1500 bis 1'000'000, gemessen durch Gelpermeationschromatographie mit Polystyrol als Standard, enthaltend, bezogen auf die Gesamtmenge der im Polymer vorhandenen Strukturelemente, 100 bis 5 Mol% des wiederkehrenden Strukturelementes der Formel XIV oder XV

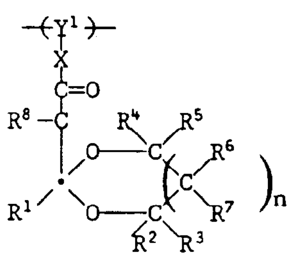

(XIV) oder

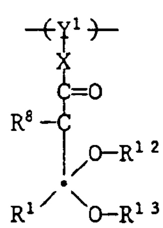

(XV)

und 95 bis 0 Mol% des wiederkehrenden Strukturelementes der Formel XVI

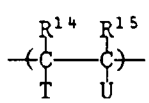

(XVI),

worin n, $R^1$, $R^2$, $R^3$, $R^4$, $R^5$, $R^6$, $R^7$, $R^8$, $R^{12}$, $R^{13}$ und X die gleiche Bedeutung wie in Formeln I und II haben und $Y^1$ für einen Rest der folgenden Formeln

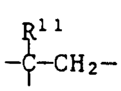 , 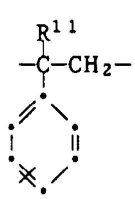 , 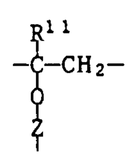 , 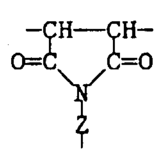 ,

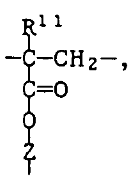 , 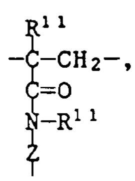 , 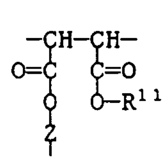 oder 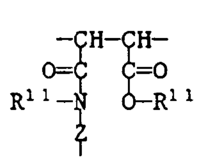 .

steht, worin $R^{11}$ und Z die gleiche Bedeutung wie oben haben, $R^{14}$ und $R^{15}$ unabhängig voneinander je ein Wasserstoffatom, unsubstituiertes oder durch Halogenatome, Cyano- oder Nitrogruppen substituiertes $C_1$-$C_4$-Alkyl oder ein unsubstituiertes oder durch Halogenatome, $C_1$-$C_4$-Alkoxy, Hydroxy-, Cyano- oder Nitrogruppen substituiertes Phenyl oder Naphthyl bedeuten, T und U unabhängig voneinander je ein Wasserstoffatom, unsubstituierte oder durch Halogenatome, Cyano- oder Nitrogruppen substituiertes $C_1$-$C_{12}$-Alkyl, unsubstituiertes oder durch Halogenatome, Hydroxy-, Cyano-, Nitrogruppen, $C_1$-$C_4$-Alkyl oder $C_1$-$C_4$-Alkoxy substituiertes Phenyl, Naphthyl oder Benzyl oder einen der folgenden Reste -$OR^{18}$, -$COOR^{19}$ oder -$COR^{20}$ bedeuten, wobei $R^{18}$ und $R^{19}$ unabhängig voneinander je ein Wasserstoffatom, unsubstituiertes oder durch Halogenatome, Cyano- oder Nitrogruppen substituiertes $C_1$-$C_{12}$-Alkyl, unsubstituiertes oder durch Halogen, Cyano-, Nitrogruppen, $C_1$-$C_4$-Alkyl oder $C_1$-$C_4$-Alkoxy substituiertes Phenyl oder Naphthyl bedeuten und $R^{20}$ die gleiche Bedeutung wie $R^{18}$ hat und ausserdem für den Rest

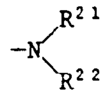

steht, worin $R^{21}$ und $R^{22}$ unab hängig voneinander die gleiche Bedeutung wie $R^{18}$ haben.

Die erfindungsgemässen polymeren β-Ketoesteracetale weisen vorzugsweise ein Molekulargewicht (Mw) von 5000 bis 500'000, insbesondere von 20'000 bis 150'000, auf.

Ferner enthalten die erfindungsgemässen polymeren β-Ketoesteracetale vorzugsweise 100 bis 20 Mol%, insbesondere 100 bis 50 Mol%, des wiederkehrenden Strukturelementes der Formel XIV oder XV und 80 bis 0 Mol%, insbesondere 50 bis 0 Mol%, des wiederkehrenden Strukturelementes der Formel XVI.

In den wiederkehrenden Strukturelementen der Formeln XIV und XV haben n, $R^1$, $R^2$, $R^3$, $R^4$, $R^5$, $R^6$, $R^7$, $R^8$, $R^{12}$, $R^{13}$ und X die gleiche bevorzugte Bedeutung wie in Formel I und II.

Der Rest $Y^1$ steht vorzugsweise für einen Rest der Formeln

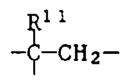 , 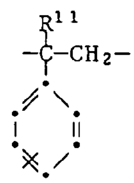 , 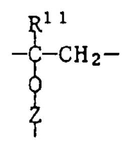 oder 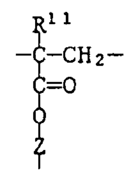 ,

wobei der zuletzt angegebene Rest besonders bevorzugt für $Y^1$ ist.

Bedeuten $R^{14}$, $R^{15}$, T und U im Strukturelement der Formel XVI ein Alkyl, so handelt es sich dabei um geradkettige oder verzweigtkettige, bevorzugt um geradkettige Alkylreste.

Halogenatome als Substituenten können Fluor, Chlor, Brom oder Jod sein und sind bevorzugt Chlor oder Brom.

Als gegebenenfalls substituiertes Alkyl kommt beispielsweise Methyl, Ethyl, 2-Chlorethyl, 2-Nitroethyl, n-Propyl, Isopropyl, n-Butyl, sek.-Butyl, tert.-Butyl, n-Pentyl, Isoamyl, n-Hexyl, 2-Ethylhexyl, n-Decyl, 6-Nitrohexyl oder 9-Bromnonyl in Frage.

Beispiele für substituiertes Phenyl oder Naphthyl sind o-, m- oder p-Chlorphenyl, o-, m- oder p-Tolyl, Xylyl, 2-Nitrophenyl oder α-Chlornaphthyl.

Die erfindungsgemässen polymeren β-Ketoesteracetale können hergestellt werden, indem man Verbindungen der Formel I oder II oder Gemische aus Verbindungen der Formel I oder II und darin bis zu 95 Mol%, vorzugsweise bis zu 80 Mol%, insbesondere bis zu 50 Mol%, enthaltenen Verbindungen der Formel XVIa

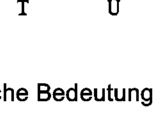 (XVIa),

worin $R^{14}$, $R^{15}$, T und U die gleiche Bedeutung wie in Formel XVI haben, in bekannter Weise radikalisch polymerisiert.

Die radikalische Polymerisation kann unter Anwendung verschiedener Techniken durchgeführt werden. Diese sind beispielsweise von S. Sandler und W. Karo in "Polymer Synthesis" Vol. 1-3, 1968, Academic Press, New York, beschrieben worden. Uebliche Polymerisationsverfahren sind beispielsweise Polymerisation in der Masse oder in Lösungsmitteln, Emulsions-, Suspensions- oder Fällungspolymerisation.

Die Verbindungen der Formel XVIa sind bekannt und zum Teil im Handel erhältlich. Neben Olefinen, wie

beispielsweise Ethylen oder Propylen, sind als Beispiele für Verbindungen der Formel XVIa insbesondere die Vinylverbindungen zu nennen. Beispiele solcher Monomere sind die Styroltypen, wie beispielsweise Styrol, α-Methylstyrol, p-Methylstyrol oder p-Hydroxyphenylstyrol, α,β-ungesättigte Säuren, deren Ester oder Amide, wie beispielsweise Acrylsäure, Acrylsäuremethylester, Acrylsäureamid, die entsprechenden Methacrylverbindungen, Maleinsäure, Maleinsäuremethylester, Maleinimide oder p-Hydroxyphenylmaleinmimide, halogenhaltige Vinylverbindungen, wie beispielsweise Vinylchlorid, Vinylfluorid, Vinylidenchlorid oder Vinylidenfluorid, Vinylester, wie beispielsweise Vinylacetat oder Vinylether, wie beispielsweise Methylvinylether oder Butylvinylether.

Weitere geeignete Verbindungen sind beispielsweise die Allylverbindungen, wie Allylchlorid, Allylbromid oder Allylcyanid.

Die Polymerisation wird in der Regel durch einen der üblichen Initiatoren der radikalischen Polymerisation eingeleitet. Dazu zählen thermische Initiatoren, wie Azoverbindungen, beispielsweise Azoisobutyronitril (AIBN), oder Peroxide, beispielsweise Benzoylperoxid, oder Redoxinitiatorsysteme, wie eine Mischung von Eisen(III)acetyl-acetonat, Benzoin und Benzoylperoxid, oder photochemische Radikalbildner, wie Benzoin oder Benzilmethylketal.

Die Copolymerisation kann in Lösung ausgeführt werden. Die Reaktionstemperatur bewegt sich im allgemeinen im Bereich von 10 bis 200°C, vorzugsweise zwischen 40 und 150°C, besonders bevorzugt zwischen 40 und 100°C.

Gegebenenfalls anwesende Lösungsmittel müssen unter den Reaktionsbedingungen inert sein. Als Lösungsmittel kommen u.a. aromatische Kohlenwasserstoffe, chlorierte Kohlenwasserstoffe, Ketone und Ether in Betracht. Beispiele dafür sind Benzol, Toluol, Xylol, Ethylbenzol, Isopropylbenzol, Ethylenchlorid, Propylenchlorid, Methylenchlorid, Chloroform, Methylethylketon, Aceton, Cyclohexanon, Diethylether oder Tetrahydrofuran.

Eine andere Möglichkeit, die erfindungsgemässen polymeren β-Ketoesteracetale herzustellen, besteht in der Acetalisierung von polymeren β-Ketoestern, die durch radikalische Polymerisation von Verbindungen der Formel III, gegebenenfalls mit den Comonomeren der Formel XVIa erhalten werden. Zur Acetalisierung können dann die Verbindungen der Formel IV bzw. der Formeln V und VI eingesetzt werden.

Wie eingangs erwähnt, stellen die erfindungsgemässen, polymeren β-Ketoesteracetale wertvolle Lösungsinhibitoren für positive Photoresists dar, die gegenüber Säuren eine sehr gute Empfindlichkeit aufweisen und zusammen mit säuregenerierenden Photoinitiatoren ein strahlungsempfindliches Gemisch bilden. Die Empfindlichkeit der erfindungsgemässen polymeren β-Ketoesteracetale gegenüber Säuren wird auch in hohen Schichtdicken, beispielsweise bei 30 μm, beibehalten. Die durch Säurespaltung aus den erfindungsgemässen polymeren Verbindungen erhaltenen polymeren β-Ketoester sind zudem basenlöslich. Dagegen sind die erfindungsgemässen polymeren β-Ketoesteracetale gegenüber Basen sehr stabil, so dass im Photoresist eine sehr gute Differenzierung zwischen belichteten und unbelichteten Stellen erhalten wird.

Die Erfindung umfasst somit auch ein strahlungsempfindliche Gemisch enthaltend, bezogen auf die Gesamtmenge der Komponenten a) und b), 85 bis 99 Gew.% eines

a) polymeren β-Ketoesteracetals mit den Strukturelementen der Formel XIV oder XV und gegebenenfalls der Formel XVI und

b) 1 bis 15 Gew.% einer unter Einwirkung von aktinischer Strahlung säurebildenden Substanz.

Vorzugsweise enthält das strahlungsempfindliche Gemisch 90 bis 99 Gew.%, insbesondere 95 bis 98 Gew.% der Komponente a) und 1 bis 10 Gew.%, insbesondere 2 bis 5 Gew.%, der Komponente b).

Als strahlungsempfindliche Komponenten b), die bei Lichteinwirkung Säure bilden bzw. abspalten, sind eine grosse Anzahl von Verbindungen bekannt. Dazu zählen beispielsweise Diazoniumsalze, wie sie in der Diazotypie verwendet werden, o-Chinondiazide, wie sie in bekannten positiv arbeitenden Kopiermassen verwendet werden, oder auch Halogenverbindungen, die bei Bestrahlung Halogenwasserstoffsäure bilden. Verbindungen dieses Typs sind beispielsweise in den US-PS 3,515,552, 3,536,489 oder 3,779,778, sowie in den DE-OSen 2,718,259, 2,243,621 oder 2,610,842 beschrieben.

Als strahlungsempfindliche Komponenten b) der erfindungsgemässen Zusammensetzungen eignen sich aber auch kationische Photoinitiatoren aus der Gruppe der Iodonium- oder Sulfoniumsalze. Solche Verbindungen sind beispielsweise in "UV-Curing, Science and Technology" (Editor: S.P. Pappas, Technology Marketing Corp., 642 Westover Road, Stanford, Connecticut, USA) beschrieben. Insbesondere sind auch Diaryliodosyl-Salze einsetzbar. Solche Verbindungen sind beispielsweise in der EP-A 106,797 beschrieben.

Ferner können Sulfoxoniumsalze als strahlungsempfindliche Verbindungen verwendet werden. Solche Salze sind beispielsweise in der EP-PS 35,969 oder in der EP-A 44,274 bzw. 54,509 beschrieben. Insbesondere zu erwähnen sind aliphatische Sulfoxoniumsalze, die im tiefen UV-Bereich absorbieren.

Insbesondere lassen sich auch Verbindungen einsetzen, die bei Bestrahlung mit aktinischem Licht Sulfonsäuren freisetzen. Solche Verbindungen sind an sich bekannt und beispielsweise in der GB-A 2,120, 263, den EP-A 84,515, 37,152 oder 58,638 bzw. in den US-PS 4,258,121 oder 4,371,605 beschrieben.

Werden als strahlungsempfindliche säureabspaltende Komponenten b) Salze eingesetzt, so sind diese vorzugsweise in organischen Lösungsmitteln löslich. Besonders bevorzugt handelt es sich bei diesen Salzen um Abscheidungsprodukte mit komplexen Säuren, beispielsweise von Borfluorwasserstoffsäure, Hexafluorphosphonsäure, Hexafluorarsensäure oder Hexafluorantimonsäure.

Den erfindungsgemässen strahlungsempfindlichen Gemischen können gegebenenfalls auch Bindemittel (c) zugesetzt werden, was besonders zweckmässig ist, wenn es sich bei den lichtempfindlichen Zusammensetzungen um flüssige oder niedrigviskose Gemische handelt oder wenn die Gemische ein homopolymeres β-Ketoesteracetal enthalten. In diesem Fall wirkt das β-Ketoesteracetal als üblicher Lösungsinhibitor. Nach dem Belichten entsteht aus dem polymeren β-Ketoesteracetal durch säurekatalysierte Hydrolyse wieder der polymere β-Ketoester, welcher basenlöslich ist und die Auflösung des basenlöslichen Binderpolymers nicht mehr behindert.

Es ist aber auch möglich, als polymeres β-Ketoesteracetal ein Copolymer einzusetzen, dass unter Verwendung von basenlöslichen Comonomeren als Verbindung der Formel XVIa hergestellt wurde. Solche Copolymere vereinen die Eigenschaften eines Lösungsinhibitors mit denen eines Bindemittels, so dass es in diesem Fall nicht unbedingt erforderlich ist, dem strahlungsempfindlichen Gemisch zusätzlich ein Bindemittel (c) zuzusetzen.

Die Menge des Bindemittels (c) kann 30-90 Gew.%, vorzugsweise 60-90 Gew.%, betragen, bezogen auf die gesamte Menge an Komponenten a), b) und c).

Die Wahl des Bindemittels erfolgt je nach dem Anwendungsgebiet und den hierfür geforderten Eigenschaften, wie Entwickelbarkeit in wässrigen und wässrig-alkalischen Lösungsmittelsystemen oder Adhäsion auf Substraten.

Geeignete Bindemittel c) sind beispielsweise Novolake, die sich von einem Aldehyd, vorzugsweise Formaldehyd, Acetaldehyd oder Furfuraldehyd, besonders jedoch von Formaldehyd, und einem Phenol ableiten. Die phenolische Komponente dieser Bindemittel ist vorzugsweise Phenol selbst, oder auch halogeniertes Phenol, beispielsweise substituiert mit ein bis zwei Chloratomen, vorzugsweise p-Chlorphenol, oder sie ist ein durch ein bis zwei $C_1$-$C_9$-Alkylgruppen substituiertes Phenol, beispielsweise o- m- oder p-Kresol, ein Xylenol, p-tert.Butylphenol oder p-Nonylphenol. Es kann sich bei der Phenolkomponente der bevorzugten Novolake aber auch um p-Phenylphenol, Resorcin, Bis-(4-hydroxyphenyl)-methan oder 2,2-Bis-(4-hydroxyphenyl)-propan handeln.

Ein Teil der phenolischen Hydroxygruppen dieser Novolake kann gegebenenfalls durch Umsetzung mit Chloressigsäure, Isocyanaten, Epoxiden oder Carbonsäureanhydriden modifiziert sein.

Weitere geeignete Bindemittel sind beispielsweise Copolymere von Maleinsäureanhydrid mit Styrol oder Vinylethern oder 1-Alkenen. Ebenfalls als Bindemittel einsetzen lassen sich:
Homo- und copolymere Acrylate und Methacrylate, z.B. Copolymere aus Methylmethacrylat/Aethylacrylat/Methacrylsäure, Poly(methacrylsäurealkylester) oder Poly(acrylsäurealkylester), wobei Alkyl = $C_1$-$C_{20}$ ist.

Vorzugsweise verwendet man als Bindemittel eine alkalilösliche Substanz, beispielsweise einen Novolak (gegebenenfalls modifiziert, wie oben beschrieben), Copolymere von Maleinsäureanhydrid mit Styrol oder Vinylethern oder 1-Alkenen, sowie Copolymere von Estern der Acrylsäure oder der Methacrylsäure mit ethylenisch ungesättigten Säuren, beispielsweise Methacrylsäure oder Acrylsäure.

Diesen alkalilöslichen Bindemitteln können gegebenenfalls noch weitere Zusatzharze beigegeben werden, wie bei den Positiv-Systemen auf Diazoketon Basis üblich. Zu diesen Zusatzharzen zählen beispielsweise Vinylpolymerisate, wie Polyvinylacetat, Polyacrylate, Polyvinylether oder Polyvinylpyrrolidone. Im allgemeinen werden jedoch nicht mehr als 20 Gew.%, bezogen auf die Menge an alkalilöslichem Bindemittel, von diesen Zusatzharzen zugefügt.

Die erfindungsgemässen Zusammensetzungen können weitere übliche Zusatzstoffe enthalten, wie z.B. Stabilisatoren, Pigmente, Farbstoffe, Füllstoffe, Haftvermittler, Verlaufmittel, Netzmittel und Weichmacher. Ferner können die Zusammensetzungen zur Applikation in geeigneten Lösungsmitteln gelöst werden.

Die erfindungsgemässen Zusammensetzungen eignen sich hervorragend als Beschichtungsmittel für Substrate aller Art, z.B. Holz, Textilien, Papier, Keramik, Glas, Kunststoffe, wie Polyester, Polyethylenterephthalat, Polyolefine oder Celluloseacetat, insbesondere in Form von Filmen, sowie Metalle, wie Al, Cu, Ni, Fe, Zn, Mg oder Co, und GaAs, Si oder $SiO_2$, bei denen durch bildmässiges Belichten eine Abbildung aufgebracht werden soll. Ein weiterer Gegenstand vorliegender Erfindung sind die beschichteten Substrate.

Die Erfindung betrifft ferner auch ein Verfahren zur Erzeugung von positiven Abbildungen umfassend folgende Arbeitsschritte:

a) Beschichtung eines Substrates mit einer strahlungsempfindlichen Zusammensetzung wie oben definiert,

b) Belichtung des beschichteten Substrates mit einem vorbestimmten Muster aktinischer Strahlung, und

c) Entwicklung des belichteten Substrates.

Die Herstellung der beschichteten Substrate kann z.B. erfolgen, indem man eine Lösung oder Suspension der Zusammensetzung herstellt.

Die Wahl des Lösungsmittels und die Konzentration richtet sich hauptsächlich nach der Art der Zusammensetzung und nach dem Beschichtungsverfahren. Die Lösung wird mittels bekannten Beschichtungsverfahren auf ein Substrat gleichförmig aufgebracht, z.B. durch Schleudern, Tauchen, Rakelbeschichtung, Vorhanggiessverfahren, Aufpinseln, Sprühen, speziell durch elektrostatisches Sprühen und Reverse-Rollbeschichtung. Es ist auch möglich, die lichtempfindliche Schicht auf einen temporären, flexiblen Träger zu bringen, und dann durch Schichtübertragung via Lamination das endgültige Substrat, z.B. eine kupferkaschierte Leiterplatte, zu beschichten.

Die Auftragsmenge (Schichtdicke) und Art des Substrates (Schichtträger) sind abhängig vom gewünschten Applikationsgebiet. Besonders vorteilhaft ist es, dass die erfindungsgemässen Zusammensetzungen in weiter variablen Schichtdicken eingesetzt werden können. Dieser Schichtdickenbereich umfasst Werte von ca. 0,5 µm bis mehr als 100 µm. Mit konventionellen Positiv-Systemen auf Naphthochinondiazid-Basis sind Schichtdicken von grösser als 10 µm nicht mehr verwendbar.

Mögliche Einsatzgebiete der erfindungsgemässen Zusammensetzungen sind die Verwendung als Photoresists für die Elektronik (Galvanoresist, Aetzresist, Lötstoppresist), die Herstellung von Druckplatten, wie Offsetdruckplatten oder Siebdruckformen, der Einsatz beim Formteilätzen oder der Einsatz als Mikroresist bei der Herstellung integrierter Schaltkreise.

Dementsprechend unterschiedlich sind die möglichen Schichtträger und die Verarbeitungsbedingungen der beschichteten Substrate.

Für photographische Informationsaufzeichnung dienen z.B. Folien aus Polyester, Celluloseacetat oder mit Kunststoff beschichtete Papiere; für Offsetdruckformen speziell behandeltes Aluminium und für die Herstellung gedruckter Schaltungen kupferkaschierte Laminate. Die Schichtdicken für photographische Materialien und Offsetdruckformen betragen ca. 0,5 µm bis 10 µm; für gedruckte Schaltungen 1 bis ca. 100 µm.

Nach dem Beschichten wird das Lösungsmittel in der Regel durch Trocknen entfernt und es resultiert eine Schicht des Photoresists auf dem Träger.

Nach der in üblicher Weise erfolgten bildmässigen Belichtung des Materials werden die belichteten Stellen des Photolackes durch Herauslösen in einem Entwickler entfernt.

Die Wahl des jeweiligen Entwicklers richtet sich nach der Art des Photolackes, insbesondere nach der Natur des verwendeten Bindemittels oder der entstehenden Photolyseprodukte. Der Entwickler kann wässrige Lösungen von Basen umfassen, denen gegebenenfalls organische Lösungsmittel oder deren Mischungen zugesetzt wurden. Bevorzugt wird die Verwendung einer wässrigen Lösung einer Base als Entwicklungsmittel. Säuren lassen sich nicht anwenden, da deren Einsatz zur Spaltung des β-Acetalesters bzw. β-Ketalesters (oder des entsprechenden Amids) an den unbelichteten Stellen des beschichteten Substrates führen würde, und somit die Ablösung der gesamten Lackschicht zur Folge hätte.

Besonders bevorzugt als Entwickler werden wässrig alkalische Lösungen, wie sie auch für die Entwicklung von Naphthochinondiazidschichten eingesetzt werden. Dazu zählen insbesondere wässrige Lösungen von Alkalimetallsilikaten, -phosphaten, -hydroxiden und -carbonaten. Diesen Lösungen können gegebenenfalls noch kleinere Mengen an Netzmitteln und/oder organischen Lösungsmitteln zugesetzt sein.

Typische organische Lösungsmittel, die den Entwicklerflüssigkeiten zugesetzt werden können, sind beispielsweise Cyclohexanon, 2-Ethoxyethanol, Toluol, Aceton, sowie Mischungen zweier oder mehrerer dieser Lösungsmittel.

Der Begriff 'Belichtung mit einem vorbestimmten Muster aktinischer Strahlung' beinhaltet sowohl die Belichtung durch eine Photomaske, die ein vorbestimmtes Muster enthält, beispielsweise ein Diapositiv, sowie die Belichtung durch einen Laserstrahl, der beispielsweise computergesteuert über die Oberfläche des beschichteten Substrates bewegt wird, und auf diese Weise ein Bild erzeugt.

Die Lichtempfindlichkeit der erfindungsgemässen Zusammensetzungen reicht in der Regel vom UV-Gebiet (ca. 200 nm) bis ca. 600 nm und umspannt somit einen sehr breiten Bereich. Als Lichtquellen kommen daher eine grosse Anzahl der verschiedensten Typen zur Anwendung. Es sind sowohl Punktlichtquellen als auch flächenförmige Strahler (Lampenteppiche) geeignet. Beispiele sind: Kohlelichtbogenlampen, Xenon-Lichtbogenlampen, Quecksilberdampflampen, gegebenenfalls mit Metall-Halogeniden dotiert (Metall-Halogenlampen), Fluoreszenzlampen, Argonglühlampen, Elektronenblitzlampen, photographische Flutlichtlampen und Röntgenstrahlen. Der Abstand zwischen Lampe und erfindungsgemässen Bildmaterial kann je nach Anwendungszweck und Lampentyp bzw. -stärke variieren, z.B. zwischen 2 cm bis 150 cm. Speziell geeignet sind Laserlichtquellen, z.B. Argonionenlaser oder Kryptonionenlaser mit starken Emissionslinien (Ar-Laser) bie 457, 476, 488, 514, 528 nm. Bei dieser Art der Belichtung ist keine Photomaske im Kontakt mit der Photopolymerschicht mehr nötig; der gesteuerte Laser-Strahl schreibt direkt auf die Schicht. Hier ist die hohe Empfindlichkeit der erfindungsgemässen Materialien sehr vorteilhaft, die hohe Schreibgeschwindigkeiten bei relativ niedrigen

Intensitäten erlaubt. Nach dieser Methode können gedruckte Schaltungen in der Elektronikindustrie, lithographische Offsetdruckplatten oder Reliefdruckplatten sowie photographische Bildaufzeichnungsmaterialien hergestellt werden.

Die lichtempfindlichen Zusammensetzungen können gegebenenfalls auch Sensibilisatoren enthalten, um die spektrale Empfindlichkeit in einem bestimmten Bereich des elektromagnetischen Spektrums zu erhöhen. Zu diesen Sensibilisatoren zählen beispielsweise Michlers Keton, Benzophenone, Thioxanthone oder aromatische Kohlenwasserstoffe wie Anthracen, Pyren oder Perylen. Speziell geeignet sind 9-Methylanthracen und 9,10-Diethoxyanthracen.

Das Bestrahlen mit Elektronenstrahlen ist eine weitere Abbildungsmöglichkeit. Elektronenstrahlen können die erfindungsgemässe Zusammensetzung durchgreifend zersetzen und auch gegebenenfalls vernetzen, so dass ein negatives Bild entsteht, wenn die unbestrahlten Teile durch Lösungsmittel oder Belichten ohne Vorlage und Entwickeln entfernt werden. Bei geringer Intensität und/oder höherer Schreibgeschwindigkeit des Elektronenstrahls bewirkt dagegen der Elektronenstrahl eine Differenzierung in Richtung höherer Löslichkeit, d.h., die bestrahlten Schichtpartien können vom Entwickler entfernt werden. Wenn das Verfahren mit Elektronenstrahlen durchgeführt wird, sind neben den bekannten für sichtbares und nahes UV-Licht empfindlichen photolytischen Säurespendern auch solche geeignet, deren Absorptionsbereiche im kürzerwelligen Teil des elektromagnetischen Spektrums liegen und die damit gegenüber Tageslicht wenig empfindlich sind. Dies hat den Vorteil, dass man die Aufzeichnungsmaterialien ohne Lichtausschluss handhaben kann und dass die Materialien besser lagerfähig gemacht werden können. Als Beispiele für derartige Starter sind Tribrommethylphenylsulfon, 2,2′,4,4′,6,6′-Hexabromdiphenylamin, Pentabromethan, 2,3,4,5-Tetrachloranilin, Pentaerythrittetrabromid, Clophen-Harz W, d.h., ein Chlorterphenylharz, oder chlorierte Paraffine zu nennen. Die erfindungsgemässen Zusammensetzungen können in den unterschiedlichsten Einsatzgebieten Verwendung finden. Sie werden insbesondere dort eingesetzt, wo eine photolithographische Abbildung mit hoher Auflösung hergestellt werden soll.

Die Erfindung betrifft daher auch die Verwendung der Zusammensetzungen, wie oben definiert, als Positiv-Photoresists für die Herstellung von Druckformen, gedruckten Schaltungen oder integrierten Schaltkreisen, sowie für silberlose photographische Filme. Ferner betrifft die Erfindung die unter Verwendung besagter Zusammensetzungen hergestellten Druckformen, gedruckten Schaltungen oder integrierten Schaltkreise oder silberlosen photographischen Filme.

Beispiel 1:

Herstellung von α(2-Methyl-1,3-dioxolanyl-2)-acetoxyethylmethacrylat [(2-Methyl-1,3-dioxolanyl-2)-8-oxo-7-oxa-4-oxa-3-oxo-2-methylnonen-1]

21,4 g (0,1 Mol) 2-Acetoacetoxyethylmethacrylat, 18,62 g (0,3 Mol) Ethylenglykol und 0,19 g (1 mMol) p-Toluolsulfonsäure-monohydrat (1 Mol% p-TsOH) werden in 200 ml Benzol gelöst und unter Rückfluss an einem Wasserabscheider gekocht. Als Polymerisationsinhibitor werden 1,7 g (5 Mol%) 2,2,4,6-Ralox® [2,2′-Methylen-bis-(4,6-di-tert.-butylphenol), Handelsprodukt der Firma Raschig GmbH,DE] zugegeben.

Nach 3 h sind 1,9 ml Wasser (theoretische Menge: 1,8 ml) abgeschieden. Das Benzol wird abdestilliert, der Rückstand wird in 150 ml Ether gelöst und zweimal mit 75 ml gesättigter Natriumcarbonatlösung und einmal mit 75 ml einer gesättigten Natriumchloridlösung extrahiert. Die vereinigten Wasserphasen werden noch einmal mit 50 ml Ether extrahiert. Die vereinigten organischen Phasen werden über $MgSO_4$ getrocknet, filtriert und am Rotationsverdampfer eingeengt. Es werden 26,2 g eines farblosen Rückstandes erhalten [gemäss Gaschromatographie (GC) 87 % Reinheit]. Der Rückstand wird einer fraktionierten Destillation am Hochvakuum bei 0,02 mbar unterworfen. Es werden 21,1 g eines farblosen Oels (Sdp.: 107-115°C/0,02 mbar; GC > 95 %), erhalten, was einer Ausbeute von 81 % der Theorie entspricht. Der Destillationsrückstand wiegt 3,8 g.

Elementaranalyse: Ber. C = 55,81 %; H = 7,03 %
Gef. C = 55,73 %; H = 7,07 %

Die Daten des IR-, $^1$H-NMR- und $^{13}$C-NMR-Spektrums bestätigen, dass eine Verbindung der Formel

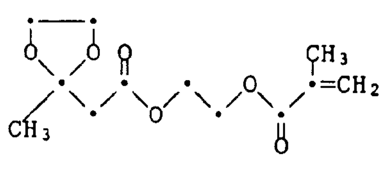

vorliegt.

Beispiel 2:

Herstellung von α-(2-Methyl-1,3-dioxanyl-2)-acetoxyethylmethacrylat

Wie in Beispiel 1 beschrieben wird aus 2-Acetoacetoxyethylmethacrylat (107,1 g) und 1,3-Propandiol (41,9 g) in Benzol (1000 ml) unter p-TsOH-Katalyse am Wasserabscheider obige Verbindung hergestellt. Ausbeute: 52 % der Theorie; GC ~ 90 % Reinheit nach Destillation an einem Dünnschichtverdampfer.

Die Daten des IR- und $^1$H-NMR-Spektrums bestätigen, dass eine Verbindung der Formel

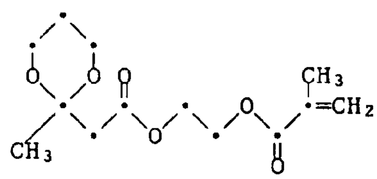

erhalten wird.

Beispiel 3:

Herstellung von α-(5-Butyl-2-methyl-1,3-dioxolanyl-2)-acetoxyethylmethacrylat

Wie in Beispiel 1 beschrieben wird aus 2-Acetoacetoxyethylmethacrylat (42,8 g) und 1,2-Hexandiol (26 g) in Benzol (200 ml) unter p-TsOH-Katalyse am Wasserabscheider obige Verbindung hergestellt. Ausbeute: 77 % der Theorie, Sdp. = 130-136°C/0,02 mbar.

| | | |
|---|---|---|
| Elementaranalyse: | Ber. C = 61,13 % | Gef. C = 61,4 % |
| | Ber. H = 8,34 % | Gef. H = 8,36 % |

Die Daten des IR- und $^1$H-NMR-Spektrums bestätigen, dass eine Verbindung der Formel

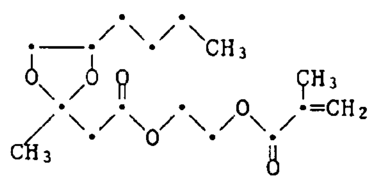

erhalten wird.

Beispiel 4:

Herstellung von α-(2,4,5-Trimethyl-1,3-dioxolanyl-2)-acetoxyethylmethacrylat

Wie im Beispiel 1 beschrieben wird aus 2-Acetoacetoxyethylmethacrylat (42,8 g) und 2,3-Butandiol (19 g) in Benzol (200 ml) unter p-TsOH-Katalyse am Wasserabscheider obige Verbindung hergestellt, Ausbeute: 63 % der Theorie; Sdp. 107-109°C/0,02 mbar. GC = 95 % Reinheit.

| | | |
|---|---|---|
| Elementaranalyse: | Ber. C = 58,73 % | Gef. C = 58,68 % |
| | Ber. H = 7,75 % | Gef. H = 7,71 % |

Die Daten des IR- und $^1$H-NMR-Spektrums bestätigen, dass eine Verbindung der Formel

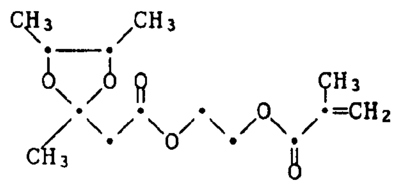

erhalten wird.

Beispiel 5:

Herstellung von α-(2-Phenyl-1,3-dioxolanyl-2)-acetoxyethylmethacrylat

a) 192,2 g (1 Mol) Benzoylessigsäureethylester und 186,2 g (3 Mol) Ethylenglykol werden in einer Destillationsapparatur innerhalb einer Stunde auf 160° erhitzt. Nach einer weiteren Stunde sind 5 ml Ethanol abdestilliert. Der Destillationsaufsatz wird gegen einen Wasserabscheider ausgetauscht und es werden 3,44 g (20 mMol) p-TsOH und 1 l Benzol zugegeben. Nach 10 Stunden Kochen unter Rückfluss sind 18 ml Wasser abgeschieden. Das Produkt wird wie in Beispiel 1 beschrieben isoliert.

Es werden nach zweimaliger Destillation am Dünnschichtverdampfer 28 g eines farblosen Oels erhalten. Ausbeute: 11 % der Theorie; GC > 95 % Reinheit.

| | | |
|---|---|---|
| Elementaranalyse: | Ber. C = 61,9 % | Gef. C = 61,69 % |
| | Ber. H = 6,39 % | Gef. H = 6,43 % |

Die Daten des IR- und $^1$H-NMR-Spektrums bestätigen, dass eine Verbindung der folgenden Formel

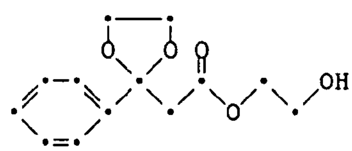

erhalten wird.

b) 63 g (0,25 Mol) der unter a) hergestellten Verbindung, 28,8 g (0,275 Mol) Methacrylsäurechlorid und 0,85 g 2,2,4,6-Ralox® werden in 400 ml Ether gelöst, und bei Raumtemperatur wird während 1 Stunde eine Lösung von 23,7 g (0,3 Mol) Pyridin in 100 ml Ether zugetropft. Nach 72 h Rühren bei Raumtemperatur wird die weisse Suspension auf Eiswasser gegossen. Die organische Phase wird zweimal mit 150 ml 1N $NaHCO_3$-Lösung gewaschen, über $MgSO_4$ getrocknet, filtriert und eingeengt. Nach Destillation am Hochvakuum werden 31 g eines farblosen Oels erhalten. Ausbeute: 39 % der Theorie, Sdp.: 142-144°/0,02 mbar; GC ~ 92 % Reinheit.

| | | |
|---|---|---|
| Elementaranalyse: | Ber. C = 63,74 % | Gef. C = 63,67 % |
| | Ber. H = 6,29 % | Gef. H = 6,36 % |

Beispiel 6:

Herstellung von α-(2-Methyl-1,3-dioxolanyl-2)-acetoxyethylmethacrylat

a) 200,3 g (2 Mol) Acetessigester und 248,3 g (4 Mol) Ethylenglykol werden in einer Destillationsapparatur bei Normaldruck auf 140°C erhitzt; nach 3 h sind 115 ml Ethanol abdestilliert (theoretische Menge: 114 ml). Es wird bei 180°C noch 2 h weiterreagieren lassen, es destillierten weitere 10 ml Ethanol über. Anschliessend wird im Vakuum 0,01 mbar fraktioniert destilliert. Es werden 129,8 g eines farblosen Oels (Sdp.: 75-85°/0,01 mbar) erhalten, was einer Ausbeute von 44 % der Theorie entspricht.

| | | |
|---|---|---|
| Elementaranalyse: | Ber. C = 49,31 % | H = 6,90 % |
| | Gef. C = 49,16 % | H = 6,87 % |

Die Daten des IR- und $^1$H-NMR-Spektrums bestätigen, dass als Verbindung 3-Oxobuttersäure-2-hydroxyethylester erhalten wird.

b) 14,6 g (0,1 Mol) der unter a) hergestellten Verbindung und 6,83 g (0,1 Mol) Ethylenglykol werden mit 5 mMol p-TSOH als Katalysator wie in Beispiel 1 acetalisiert. Ausbeute 62 % (der Theorie); Sdp.: 85-91°/0,03 mbar; GC ~ 93 % Reinheit.

| | | | |
|---|---|---|---|
| Elementaranalyse: | Ber. | C = 50,52 % | H = 7,42 % |
| | Gef. | C = 50,65 % | H = 7,41 % |

Die Daten des IR- und $^1$H-NMR-Spektrums bestätigen, dass eine Verbindung der Formel

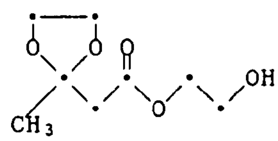

erhalten wird.

c) 9,5 g (0,05 Mol) der unter b) hergestellten Verbindung und 5,6 g (0,055 Mol) Triethylamin werden in 200 ml Diethylether gelöst. Bei Raumtemperatur (RT) werden innerhalb 20 Minuten 5,75 g (0,055 Mol) Methacrylsäurechlorid, gelöst in 100 ml Diethylether, zugetropft und die Reaktionslösung wird 1 Stunde bei Rückflusstemperatur des Ethers weitergerührt. Es wird über basisches Alox (Aluminiumoxid) filtriert und das Filtrat eingeengt, wobei 4,3 g eines Oels erhalten wird, das gemäss GC zu 76 % aus reinem α-(2-Methyl-1,3-dioxolanyl-2)-acetoxyethylmethacrylat besteht.

Beispiel 7:

Herstellung von α-(2,2-Dimethoxyethyl)-acetoxyethylmethacrylat

Acetoacetoxyethylmethacrylat wird in Methanol gelöst und wie von F. Gasparrini et al. in Tetrahedron 1984, 40, 1491-1500 beschrieben mit modifiziertem Silicagel versetzt und bei Raumtemperatur gerührt. Der Katalysator wird abfiltriert und der Rückstand eingeengt. Das Produkt wird chromatographisch gereinigt. Die Ausbeute an reinem Produkt beträgt 65 % der Theorie; GC = 92 % Reinheit. Als Nebenprodukt wird der entsprechende Enolether isoliert.

| | | | |
|---|---|---|---|
| Elementaranalyse: | Ber. | C = 55,37 % | H = 7,75 % |
| | Gef. | C = 55,58 % | H = 7,75 % |

Die Daten des IR- und $^1$H-NMR-Spektrums bestätigen, dass eine Verbindung der Formel

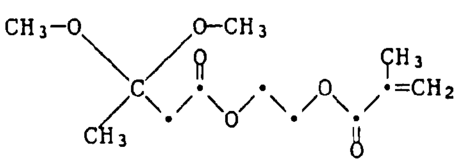

erhalten wird.

Herstellung von Polymeren bzw. Copolymeren aus den erfindungsgemässen Monomeren der Formeln I und II

Zu einer 20 %-Lösung des Monomeren in Tetrahydrofuran wird in einem Polymerisationsrohr 0,5 Gew.% α,α'-Azoisobutyronitril gegeben. Die Lösung wird bei 0°C während 30 Sekunden auf 30 mbar evakuiert und mit Argon geflutet. Das Prozedere wird dreimal wiederholt. Nachher wird das Polymerisationsrohr verschlossen und bei 65°C wird während 24 h polymerisiert. Das Polymer wird durch Ausfällen isoliert und falls nötig durch Umfällen gereinigt. Nach Trocknung wird das Polymer durch Gelpermeationschromatographie (GPC) mit Poly-

styrol als Standard, IR-, $^1$H-NMR-Spektrum und Elementaranalyse charakterisiert. Resultate sind in Tabelle 1 zusammengestellt.

Tabelle 1

| Beispiel | Monomer gemäss Beispiel | Comonomer | Mw | Mn | n Mw/Mn | Berechnet C | Berechnet H | Gefunden C | Gefunden H |
|---|---|---|---|---|---|---|---|---|---|
| A | 100 % 1 | - | 81600 | 24500 | 3,3 | 55,81 | 7,02 | 55,66 | 7,09 |
| B | 88 % 1 | 12 % MAS x) | 89100 | 30300 | 2,9 | 55,81 | 7,03 | 55,64 | 7,06 |
| C | 90 % 1 | 10 % MAS | 142700 | 24200 | 5,9 | 55,81 | 7,03 | 55,70 | 7,19 |
| D | 100 % 2 | - | 84800 | 26800 | 3,2 | 57,34 | 7,40 | 57,17 | 7,38 |
| E | 40 % 2 | 60 % AA xx) | 113000 | 25600 | 4,4 | 56,65 | 6,96 | 56,22 | 7,03 |
| F | 25 % 2 | 75 % AA | 72800 | 21000 | 3,5 | 56,44 | 6,83 | 56,19 | 6,77 |
| G | 10 % 2 | 90 % AA | 81000 | 18900 | 4,3 | 56,23 | 6,69 | 55,93 | 6,87 |
| H | 100 % 3 | - | 85000 | 14000 | 6 | 61,13 | 8,34 | 60,96 | 8,26 |
| I | 100 % 4 | - | 79300 | 13400 | 5,9 | 58,73 | 7,75 | 58,58 | 7,66 |
| J | 100 % 5 | - | 72100 | 15600 | 4,6 | 63,74 | 6,29 | 63,57 | 6,41 |

x) MAS = Methacrylsäure

xx) AA = Acetoacetoxymethylmethacrylat

## Applikationsbeispiele

### Beispiel I

Zur Herstellung von Beschichtungslösungen werden die in Tabelle 2 angegebenen Komponenten in 1-Acetoxy-2-ethoxyethan gelöst, so dass eine 30 Gew.%-ige Lösung erhalten wird. Ausser dem lichtvernetzbaren Polymer und einem Binder enthält die Beschichtungslösung, bezogen auf die Menge an Polymer und Binder, 5 Gew.% Photoinitiator, 1 Gew.% Sensibilisator ($A_1$ = 9-Methylanthracen, B = 9,10-Diethoxyanthracen), 0,5 Gew.% Netzmittel Fluorad® FC 430 (3M) und 0,2 Gew.% des Farbstoffes Orasolrot®B (Ciba-Geigy AG).

Eine gereinigte kupferkaschierte Leiterplatte wird mit Hilfe eines Ziehrakels mit der Beschichtungslösung beschichtet; Nassfilmdicke 100 µm. Die Platten werden während 30′ bei 80°C getrocknet, es resultiert eine Schichtdicke von 25-30 µm, gemessen mit einem Isoscope MP (Helmut Fischer Elektronik und Messtechnik AG). Man belichtet die beschichteten Platten durch einen Stoufferresolution und -sensitivity guide (Inkremente der optischen Dichte 0,15). Als Lichtguelle wird eine 5000 W Metall-Halogenlampe (Sylvania M061) verwendet. Der Abstand zum Vakuumrahmen beträgt 65 cm. Die Belichtungszeit wird in Impulsen des Staub-Belichtungsmessers angegeben. Die belichteten Platten werden mit dem entsprechenden Entwickler durch schwaches Reiben mit einem Wattebausch entwickelt.

Entwickler $A_2$: 75 g Natriummetasilikat·$5H_2O$
0,4 g Supronic®B50 (ABM Chemicals Ltd., Stockport, Cheshire SK6 1PQ/GB)
925 g deionisiertes Wasser.

$B_1$: Entwickler A und Wasser (1:1).

Tabelle 2

| Polymer/ Gew.% | Binder/ Gew.% | Photo-initia-tor | Sensi-bilisa-tor | Belich-tungs-zeit (Impulse) | Entwickler/ Entwick-lungszeit (Sekunden) | Keilstufen | | Qualität des Bildes |
|---|---|---|---|---|---|---|---|---|
| | | | | | | angegriffene | entwickelte | |
| A/30 | Novolak[1)]/70 | a | $A_1$ | 120 | $A_2$/420 | 4 | 3 | ++ |
| | | | | 240 | $A_2$/420 | 10 | 9 | ++ |
| A/25 | Novolak/75 | a | $A_1$ | 60 | $A_2$/120 | 4 | 3 | ++ |
| | | | | 120 | $A_2$/120 | 7 | 6 | ++ |
| | | | | 240 | $A_2$/120 | 9 | 8 | ++ |
| A/20 | Novolak/80 | a | $A_1$ | 60 | $A_2$/90 | 6 | 4 | ++ |
| | | | | 120 | $A_2$/90 | 11 | 9 | + |
| | | | | 240 | $A_2$/90 | 12 | 10 | +/- |

1) Alnovol PN 430 (Fa. Hoechst)

a) 2-(4-Methoxystyryl)-4,6-bis(trichlormethyl)-s-triazin

++ = sehr gut

\+ = gut

\- = schlecht

Beispiel II

Mischungen aus Polymer A und Novolak in einem Mischungsverhältnis von jeweils 25 zu 75 Gew.% werden mit verschiedenen Photoinitiatoren versehen, wobei die folgenden eingesetzt werden.

Photoinitiator:

a) 2-(4-Methoxystyryl)-4,6-bis(trichlormethyl)-s-triazin

b) 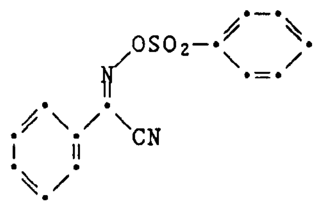

c) $Ph_3S^+PF_6^\ominus$ (Triphenylsulfonium-hexafluorophosphat)

d) 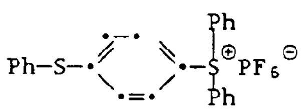 ("UVI®6974" von General Electric)

e) $Ph_3S^+SbF_6^-$ (Triphenylsulfonium-hexafluoroantimonat)
f) $Ph_2J^+PF_6^\ominus$ (Diphenyljodonium-hexafluorophosphat)
g) $Ph_2J^+AsF_6^-$ (Diphenyljodonium-hexafluoroarsenat)
h) $Ph_2J^+SbF_6^-$ (Diphenyljodonium-hexafluoroantimonat)

i) 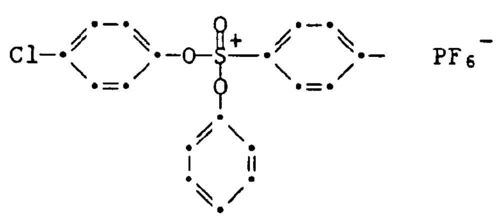

Die Eigenschaften der aus den lichtempfindlichen Mischungen hergestellten Bilder sind in Tabelle 3 angegeben. Die in Klammern angegebenen Werte werden erhalten, indem die Platte nach der Belichtung während 30 Minuten bei 40°C getempert wird.

Tabelle 3

| Polymer/ Gew.% | Binder/ Gew.% | Photo-initia-tor | Sensi-bilisa-tor | Belich-tungs-zeit (Impulse) | Entwickler/ Entwick-lungszeit (Sekunden) | Keilstufen | | Qualität des Bildes |
|---|---|---|---|---|---|---|---|---|
| | | | | | | ange-griffene | ent-wickelte | |
| A/25 | Novolak/75 | a | $A_1$ | 60<br>120 | $A_2$/90<br>$A_2$/90 | 4<br>7 | 3<br>6 | ++<br>++ |
| A/25 | Novolak/75 | b | $A_1$ | 120 | $A_2$/90 | 6(7) | 3(5) | ++ |
| A/25 | Novolak 75 | c | $A_1$ | 60<br>120 | $A_2$/90<br>$A_2$/90 | 5<br>9 | 4<br>7 | ++<br>++ |
| A/25 | Novolak/75 | d | $A_1$ | 30<br>60<br>120 | $A_2$/90<br>$A_2$/90<br>$A_2$/90 | -(1)<br>-(7)<br>3 | -(2)<br>-(5)<br>5 | /++<br>/++<br>++/ |
| A/25 | Novolak/75 | e | $A_1$ | 30<br>60 | $A_2$/90<br>$A_2$/90 | -(10)<br>11(11) | -(4)<br>4(5) | /+<br>+/+ |
| A/25 | Novolak/75 | f | $A_1$ | 30<br>60<br>120 | $A_2$/90<br>$A_2$/90<br>$A_2$/90 | 5<br>6(11)<br>13(11) | 3<br>5(9)<br>11 | ++/<br>++/+<br>+/- |
| A/25 | Novolak/75 | g | $A_1$ | 10<br>30<br>60 | $A_2$/90 | -(5)<br>5(6)<br>8(9) | -(2)<br>3(4)<br>5(7) | /++<br>++/++<br>++/++ |

Tabelle 3 (Fortsetzung)

| Polymer/ Gew.% | Binder/ Gew.% | Photo-initiator | Sensibilisator | Belichtungszeit (Impulse) | Entwickler/ Entwicklungszeit (Sekunden) | Keilstufen | | Qualität des Bildes |
|---|---|---|---|---|---|---|---|---|
| | | | | | | angegriffene | entwickelte | |
| A/25 | Novolak/75 | h | $A_1$ | 10 | $A_2$/90 | -(6) | -(4) | /+ |
| | | | | 30 | $A_2$/90 | 10(10) | 7(8) | +/+ |
| A/25 | Novolak/75 | i | $A_1$ | 15 | $A_2$/90 | 6(5) | 1(1) | ++/++ |
| | | | | 30 | $A_2$/90 | 8(8) | 4(5) | ++/++ |
| | | | | 60 | $A_2$/90 | 10(15) | 5(10 | ++/+ |
| A/25 | Novolak/75 | k | B | 5 | $A_2$/90 | 8(9) | 2(3) | +/+ |
| | | | | 10 | $A_2$/90 | 8(6) | 3(3) | +/++ |
| | | | | 30 | $A_2$/90 | 8(10) | 5(7) | ++/++ |

Beispiel III

Unter Verwendung von Polymer D und bestimmten Photoinitiatoren werden wie in Beispiel I lichtempfindliche Mischungen hergestellt und damit kupferkaschierte Platten beschichtet. Nach einer Belichtung von 90 Sekunden werden die Platten 30 Minuten bei 40°C getempert. Die Eigenschaften der erhaltenen Bilder sind in Tabelle 4 angegeben.

Tabelle 4

| Polymer/ Gew.% | Binder/ Gew.% | Photoinitiator | Sensibilisator | Belichtungszeit (Impulse) | Entwickler | Keilstufen angegriffene | Keilstufen entwickelte | Qualität des Bildes |
|---|---|---|---|---|---|---|---|---|
| D/25 | Novolak/75 | a | B | 1 | $B_1$ | 2 | 0 | - |
| | | | | 5 | $B_1$ | 5 | 2 | ++ |
| | | | | 10 | $B_1$ | 9 | 5 | ++ |
| | | | | 30 | | 14 | 10 | ++ |
| D/25 | Novolak 75 | b | B | 10 | $B_1$ | 10 | 4 | + |
| | | | | 30 | $B_1$ | 11 | 6 | + |
| D/25 | Novolak/75 | c | B | 5 | $B_1$ | 0 | 0 | |
| | | | | 10 | $B_1$ | 4 | 1 | + |
| | | | | 30 | $B_1$ | 10 | 7 | + |
| D/25 | Novolak/75 | f | $A_1$ | 1 | $B_1$ | 0 | 0 | - |
| | | | | 10 | $B_1$ | 9 | 6 | ++ |
| | | | | 30 | $B_1$ | 12 | 9 | ++ |
| D/25 | Novolak/75 | f | B | 1 | $B_1$ | 4 | 1 | + |
| | | | | 10 | $B_1$ | 8 | 4 | ++ |
| | | | | 30 | $B_1$ | 11 | 8 | ++ |
| D/25 | Novolak/75 | g | B | 1 | $B_1$ | 5 | 1 | ++ |
| | | | | 10 | $B_1$ | 10 | 6 | ++ |
| | | | | 30 | $B_1$ | 11 | 8 | ++ |

Tabelle 4 (Fortsetzung)

| Polymer/ Gew.% | Binder/ Gew.% | Photo-initiator | Sensi-bilisator | Belich-tungs-zeit (Impulse) | Entwickler | Keilstufen | | Qualität des Bildes |
|---|---|---|---|---|---|---|---|---|
| | | | | | | ange-griffene | ent-wickelte | |
| D/25 | Novolak/75 | h | B | 1 | $B_1$ | 6 | 2 | ++ |
| | | | | 10 | $B_1$ | 9 | 7 | ++ |
| | | | | 30 | $B_1$ | 12 | 10 | ++ |
| D/25 | Novolak/75 | i | B | 2 | $B_1$ | 6 | 2 | ++ |
| | | | | 10 | $B_1$ | 15 | 7 | ++ |
| | | | | 30 | $B_1$ | 14 | 10 | ++ |

Beispiel IV

Unter Verwendung von verschiedenen Polymeren werden wie in Beispiel I lichtempfindliche Beschichtungslösungen hergestellt, die jeweils 5 Gew.% Photoinitiator f und 1 Gew.% Sensibilisator $A_1$ enthalten. Nach dem Beschichten von kupferkaschierten Platten mit diesen Beschichtungslösungen werden die Platten wie in Beispiel I belichtet und entwickelt. Die Eigenschaften der erhaltenen Bilder sind in Tabelle 5 angegeben.

Tabelle 5

| Polymer/ Gew.% | Binder/ Gew.% | Belichtungszeit (Impulse) | Entwickler | Keilstufen | | Qualität des Bildes |
|---|---|---|---|---|---|---|
| | | | | angegriffene | entwickelte | |
| A/25 | Novolak/75 | 30 | $A_1$ | 5 | 3 | ++ |
| | | 60 | $A_1$ | 6 | 5 | ++ |
| | | 120 | $A_1$ | 13 | 11 | ++ |
| B/25 | Novolak 75 | 30 | $B_1$ | 5 | 3 | ++ |
| | | 60 | $B_1$ | 8 | 6 | ++ |
| | | 120 | $B_1$ | 12 | 10 | ++ |
| C/25 | Novolak/75 | 30 | $B_1$ | 3 | 1 | ++ |
| | | 60 | $B_1$ | 6 | 4 | ++ |
| | | 120 | $B_1$ | 11 | 9 | ++ |
| D/25 | Novolak/75 | 10 | $B_1$ | 9 | 6 | ++ |
| | | 30 | $B_1$ | 12 | 9 | ++ |
| | | 60 | $B_1$ | >15 | >15 | + |
| I/25 | Novolak/75 | 30 | $B_1$ | 2 | 0 | - |
| | | 60 | $B_1$ | 4 | 1 | ++ |
| | | 120 | $B_1$ | 8 | 5 | ++ |
| J/25 | Novolak/75 | 30 | $A_1$ | 6 | 4 | ++ |
| | | 60 | | 9 | 6 | ++ |

Beispiel V

Aus Polymer A wird mit verschiedenen alkalilöslichen Bindern, d.h., Polymer mit entweder phenolischen Hydroxylgruppen oder seitenständigen β-Ketoestergruppen, wie in Beispiel I lichtempfindlichen Beschichtungslösungen hergestellt, die alle Diphenyljodonium-hexafluorophosphat als Photoinitiator enthalten. Die Beschichtungslösungen werden wie in Beispiel I zu Bildern verarbeitet, deren Eigenschaften in Tabelle 6 angegeben sind. Die folgenden Binder werden eingesetzt:

Binder II: "Resin M" mit einem $M_w$ von etwa 6000, erhältlich im Handel von der Firma Maruzen Oil Co.

BinderIII: "Copolymer aus 4-Hydroxyphenylmaleinimid und Butylvinylether im Molverhältnis 1:1, hergestellt nach der Vorschrift von S.R. Turner et al in Polymer Eng. Science 26 (1986) Seiten 1096 bis 1100.

Binder IV: Poly-(2-acetoacetoxyethylmethacrylat).

Als Entwickler $C_1$ wird ein Gemisch aus 95 Teilen einer Lösung aus 112 g Natriummetasilikat·$5H_2O$, 3 g Strontiumhydroxid·$8H_2O$, 30 g Polyethylenglykol (15000) und 5 g Lävulinsäure in 10 Liter Wasser und 5 Teilen 1N NaOH eingesetzt.

Tabelle 6

| Polymer/ Gew.% | Binder/ Gew.% | Belichtungszeit (Impulse) | Entwickler | Keilstufen | | Qualität des Bildes |
|---|---|---|---|---|---|---|
| | | | | angegriffene | entwickelte | |
| A/50 | II/50 | 10 | $B_1$ | 2 | 0 | - |
| | | 30 | $B_1$ | 4 | 3 | ++ |
| | | 60 | $B_1$ | 7 | 5 | ++ |
| | | 120 | $B_1$ | 9 | 7 | ++ |
| A/40 | II/60 | 10 | $B_1$ | 4 | 1 | + |
| | | 30 | $B_1$ | 6 | 4 | ++ |
| | | 60 | $B_1$ | 9 | 6 | ++ |
| | | 120 | $B_1$ | 12 | 8 | ++ |
| A/30 | II/70 | 10 | $B_1$ | 14 | 4 | + |
| | | 30 | $B_1$ | 10 | 7 | ++ |
| | | 60 | $B_1$ | 14 | 10 | ++ |
| | | 120 | $B_1$ | 13 | 8 | ++ |
| A/25 | II/75 | 10 | $B_1$ | 6 | 1 | ++ |
| | | 30 | $B_1$ | 11 | 6 | ++ |
| | | 60 | $B_1$ | 10 | 6 | ++ |
| | | 120 | $B_1$ | 14 | 10 | + |

Tabelle 6 (Fortsetzung)

| Polymer/ Gew.% | Binder/ Gew.% | Belichtungszeit (Impulse) | Entwickler | Keilstufen | | Qualität des Bildes |
|---|---|---|---|---|---|---|
| | | | | angegriffene | entwickelte | |
| A/10 | II/90 | 10 | $B_1$ | 4 | 1 | + |
| | | 30 | $B_1$ | 6 | 4 | ++ |
| | | 60 | $B_1$ | 9 | 6 | ++ |
| | | 120 | $B_1$ | 12 | 8 | + |
| A/40 | III/60 | 10 | $C_1$ | 6 | 0 | - |
| | | 30 | $C_1$ | 9 | 1 | + |
| | | 30 | $B_1$ | 10 | 4 | ++ |
| | | 60 | $C_1$ | 11 | 5 | ++ |
| | | 120 | $C_1$ | 14 | 6 | ++ |
| A/30 | III/70 | 10 | $C_1$ | 4 | 0 | - |
| | | 30 | $C_1$ | 9 | 2 | ++ |
| | | 60 | $C_1$ | 13 | 4 | ++ |
| | | 120 | $C_1$ | 15 | 7 | ++ |
| A/25 | III/75 | 10 | $B_1$ | 7 | 0 | - |
| | | 30 | $B_1$ | 9 | 2 | ++ |
| | | 60 | $B_1$ | 10 | 4 | ++ |
| | | 120 | $B_1$ | 14 | 7 | ++ |
| A/20 | III/80 | 10 | $B_1$ | 7 | 2 | ++ |
| | | 30 | $B_1$ | 9 | 4 | ++ |
| | | 60 | $B_1$ | 12 | 6 | ++ |
| | | 120 | $B_1$ | 14 | 8 | ++ |
| A/50 | IV/50 | 60 | $B_1$ | 16 | 3 | - |
| | | 120 | $B_1$ | 5 | 3 | ++ |
| | | 120 | $C_1$ | 7 | 5 | ++ |
| | | 240 | $C_1$ | 16 | 10 | + |

Beispiel VI

Es wird eine 30%-ige Lösung in Ethylglykolacetat mit folgenden Komponenten hergestellt;

50 Teile Binder I (Novolak gemäss Beispiel I)
50 Teile Polymer A (Tabelle 1)
5 Teile Photoinitiator f
1 Teil Sensibilisator B
0,5 Teile Netzmittel Fluorad® FC 430
0,2 Teile Orasolblau® (der Firma Ciba-Geigy AG)

Eine kupferkaschierte Leiterplatte (35 µm Cu) wird mit der Lösung, wie in Beispiel I beschrieben, beschichtet und während 60 Impulse (etwa 12 Sekunden) mit einem Leiterplattenmuster belichtet. In einem Convac-Sprühentwickler wird mit dem Entwickler $B_1$ während 25 Sekunden entwickelt und anschliessend in einer 15%-igen Natriumpersulfat-Lösung während 15 Minuten bei 40°C das freigelegte Kupfer weggeätzt. Die Oberfläche des

Resists auf der Oberfläche der Leiterbahnen zeigt unter dem Mikroskop keine sichtbaren Veränderungen. Durch erneutes Belichten und Entwickeln werden die Leiterbahnen freigelegt.

Eine andere Platte wird nach dem Entwickeln in einem Glanzkupferbad der Firma Erné während 90 Minuten galvanisiert. Es werden 10 µm Cu abgeschieden. Der Resist zeigt nach der Galvanisierung unter dem Mikroskop keine sichtbaren Veränderungen. Nach erneuter Belichtung kann der Resist im Entwickler $B_1$ abgelöst werden.

Beispiel VII

Wie im Beispiel VI beschrieben ist, werden Platten mit der folgenden Lösung beschichtet, geätzt und galvanisiert:

| | | |
|---|---|---|
| 50 | Teile | Binder III |
| 50 | Teile | Polymer A |
| 5 | Teile | Photoinitiator f |
| 1 | Teil | Sensibilisator B |
| 0,5 | Teile | Netzmittel Fluorad® FC 430 |
| 0,2 | Teile | Orasolrot®B |

Die entwickelte Platte wird während 20 Minuten bei 40°C in einer 15%-igen Natriumpersulfat-Lösung geätzt. Die Oberfläche des Resists zeigt unter dem Mikroskop keine sichtbaren Veränderungen. Nach erneutem Belichten und Entwickeln konnte der Resist mit dem Entwickler $C_1$ abgelöst werden.

Eine andere Platte wird bei 40°C während 105 Minuten galvanisiert; es wird 10 µm Kupfer abgeschieden. Der Resist zeigt keine Veränderung der Oberfläche. Nach erneuter Belichtung und Entwicklung kann der Resist mit Entwickler E abgelöst werden.

Beispiel VIII

Eine 10%-ige Lösung in Ethylglykolacetat wird auf einen Si-Wafer aufgeschleudert; es resultiert eine Schichtdicke von 1,2 µm, gemessen mit einem "Alpha Stepper 100" der Firma Tencor.

| | | | |
|---|---|---|---|
| Formulierung: | 75 | Teile | Binder I |
| | 25 | Teile | Polymer A |
| | 5 | Teile | Photoinitiator f |
| | 1 | Teil | Sensibilisator B |
| | 0,5 | Teile | Netzmittel "FC 430". |

Je ein Wafer wird während 50, 100, 200 bzw. 400 Sekunden durch eine Testmaske in einer Oriel-500W-Quecksilber-Xenonlampe belichtet. Die belichteten Wafer werden während 15 Minuten bei 40°C und 1 Stunde bei 20°C stehen lassen. Durch Eintauchen der Wafer in den Entwickler $B_1$ werden die Wafer entwickelt. Alle 4 Wafer zeigen sauber entwickelte Strukturen mit steilen Flanken bis zu einer Grösse von 1,5 µm.

## Patentansprüche

1. Verbindungen der Formel I oder II

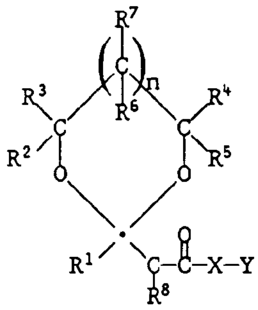

(I)

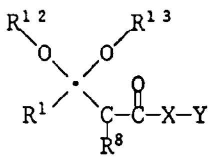

(II),

worin n für Null oder die Zahl 1 oder 2 steht,
$R^1$ ein Wasserstoffatom, ein unsubstituiertes oder durch Halogenatome substituiertes $C_1$-$C_{10}$-Alkyl, ein durch Ethersauerstoffatome unterbrochenes $C_2$-$C_{10}$-Alkyl oder ein unsubstituiertes oder durch Halogenatome, Nitrogruppen, $C_1$-$C_4$-Alkyl oder $C_1$-$C_4$-Alkoxy substituiertes Phenyl oder Benzyl bedeutet,
$R^2$, $R^3$, $R^4$, $R^5$, $R^6$, $R^7$ und $R^8$ unabhängig voneinander je ein Wasserstoffatom, Halogenatom, unsubstituiertes oder durch Halogenatome substituiertes $C_1$-$C_{10}$-Alkyl, unsubstituiertes oder durch Halogenatome, Cyano-, Nitrogruppen oder $C_1$-$C_4$-Alkyl substituiertes Phenyl oder Naphthyl oder einen Rest der Formeln -$COOR^9$,

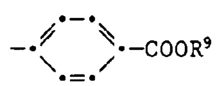

oder -$SO_2R^9$ bedeuten, worin
$R^9$ ein unsubstituiertes oder durch Halogenatome oder Nitrogruppen substituiertes $C_1$-$C_6$-Alkyl oder Phenyl bedeutet,
X für O, S oder $NR^{10}$ steht, wobei $R^{10}$ ein Wasserstoffatom, ein unsubstituiertes oder durch Halogenatome oder Nitrogruppen substituiertes $C_1$-$C_6$-Alkyl oder Phenyl bedeutet, und
Y für einen Rest der folgenden Formeln

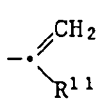 , 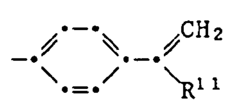 , 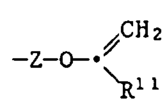 , 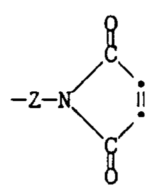 , 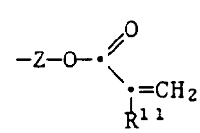 ,

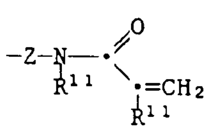 , 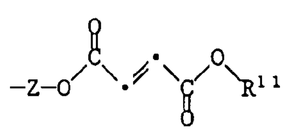 oder 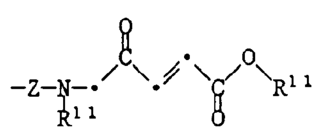

steht, worin $R^{11}$ ein Wasserstoffatom, ein unsubstituiertes oder durch Halogenatome oder Nitrogruppen substituiertes $C_1$-$C_6$-Alkyl oder Phenyl bedeutet,
Z für einen unsubstituierten oder durch Halogenatome, Nitrogruppen oder Phenyl substituierten, mindestens zwei Methylengruppen enthaltenden aliphatischen Rest oder einen unsubstituierten oder durch Halogenatome oder Nitrogruppen substituierten, mindestens zwei Methylengruppen enthaltenden, durch Ethersauerstoffatome, Phenylen oder Cyclohexylengruppen unterbrochenen aliphatischen Rest steht,
$R^{12}$ und $R^{13}$ unabhängig voneinander je ein unsubstituiertes oder durch Halogenatome oder Nitrogruppen substituiertes $C_1$-$C_{10}$-Alkyl, Phenyl oder Naphthyl bedeuten.

2. Verbindungen der Formel I oder II gemäss Anspruch 1, worin n für Null oder die Zahl 1 steht, $R^1$ ein Wasserstoffatom, ein unsubstituiertes oder durch Chloratom(e) substituiertes $C_1$-$C_4$-Alkyl, ein durch Ethersauerstoffatom(e) unterbrochenes $C_2$-$C_4$-Alkyl, Phenyl oder Benzyl bedeutet, $R^2$, $R^3$, $R^4$, $R^5$, $R^6$ und $R^7$ unabhängig voneinander je ein Wasserstoffatom, Chloratom, $C_1$-$C_6$-Alkyl oder Phenyl bedeuten, $R^8$ ein Wasserstoffatom, Phenyl, p-Nitrophenyl, p-Cyanophenyl oder den Rest -$COOR^9$ bedeutet, worin $R^9$ für ein $C_1$-$C_6$-Alkyl oder Phenyl steht, X für ein Sauerstoffatom steht,
Y für einen Rest der Formeln

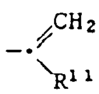 , 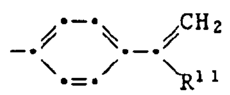 , 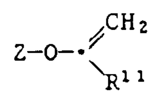 oder

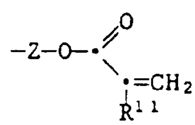

steht, worin $R^{11}$ ein Wasserstoffatom oder ein $C_1$-$C_4$-Alkyl bedeutet, Z für einen der folgenden Reste

$-(CH_2)_b-$ ,

$-CH_2-CH_2-(OCH_2-CH_2)_c-OCH_2-CH_2-$ , $-CH_2-CHR^{11}-$ , 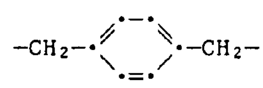

oder 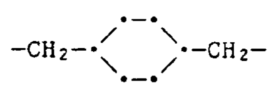

steht, worin b eine Zahl von 3 bis 5 und c Null oder eine Zahl von 1 bis 3 bedeuten, $R^{12}$ und $R^{13}$ unabhängig voneinander je ein $C_1$-$C_4$-Alkyl bedeuten.

3. Verbindungen der Formel I gemäss Anspruch 1, worin n für Null oder die Zahl 1 steht, $R^1$ ein $C_1$-$C_4$-Alkyl, Methoxymethyl oder Phenyl bedeutet, $R^2$, $R^3$, $R^4$, $R^5$, $R^6$, $R^7$ und $R^8$ je ein Wasserstoffatom bedeuten, X für ein Sauerstoffatom steht und Y den Rest

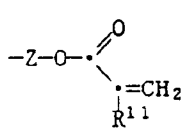

bedeutet, worin Z für Ethylen und $R^{11}$ für ein Wasserstoffatom oder Methyl stehen.

4. Verbindungen der Formel II gemäss Anspruch 1, worin $R^1$, $R^{12}$ und $R^{13}$ unabhängig voneinander je ein $C_1$-$C_4$-Alkyl bedeuten, $R^8$ für ein Wasserstoffatom steht, X für ein Sauerstoffatom steht und Y einen Rest der Formel

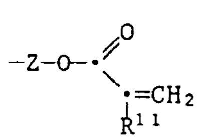

bedeutet, worin Z für Ethylen und $R^{11}$ für ein Wasserstoffatom oder Methyl steht.

5. Verfahren zur Herstellung von Verbindungen der Formel I oder II gemäss Anspruch 1, dadurch gekennzeichnet, dass man

a) eine Verbindung der Formel III

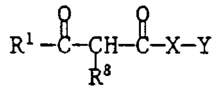 (III),

worin $R^1$, $R^8$, X und Y die gleiche Bedeutung wie in Formel I haben, entweder mit einer Verbindung der Formel IV

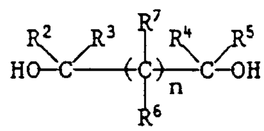 (IV),

worin n, $R^2$, $R^3$, $R^4$, $R^5$, $R^6$ und $R^7$ die gleiche Bedeutung wie in Formel I haben, zu einer Verbindung der Formel I umsetzt, wobei man auf 1 Mol der Verbindung der Formel III mindestens 1 Mol einer Verbindung der Formel IV einsetzt, oder mit Verbindungen der Formeln V und VI

$HO\text{-}R^{12}$ (V)

und

$OH\text{-}R^{13}$ (VI),

worin $R^{12}$ und $R^{13}$ die gleich Bedeutung wie in Formel II haben, zu einer Verbindung der Formel II umsetzt, wobei man auf 1 Mol der Verbindung III mindestens 2 Mol der Verbindungen der Formeln V and VI einsetzt,

b) Verbindungen der Formel VII oder VIII

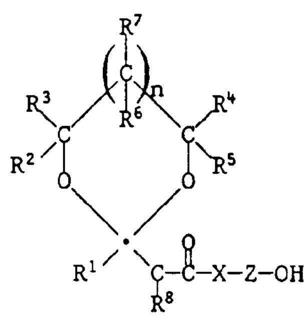 (VII)

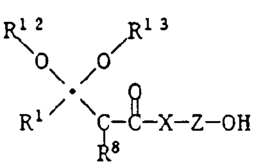 (VIII),

worin n, $R^1$, $R^2$, $R^3$, $R^4$, $R^5$, $R^6$, $R^7$, $R^8$, $R^{12}$, $R^{13}$, X und Z die gleiche Bedeutung wie in Formeln I und II haben, mit einer Verbindung der Formel IX, X oder XI

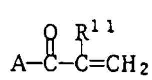 (IX)

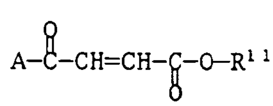 (X) oder

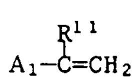 (XI),

worin $R^{11}$ die gleiche Bedeutung wie in Formel I oder II hat, A für ein Halogenatom oder eine Hydroxylgruppe steht, oder wobei die Verbindungen der Formeln IX und X auch als Anhydrid vorliegen können, und $A_1$ für ein Halogenatom oder ein $C_1$-$C_4$-Alkoxy steht, in molaren Mengen zu Verbindungen der Formel I oder II umsetzt, oder indem man

c) Verbindungen der Formel XII oder XIII

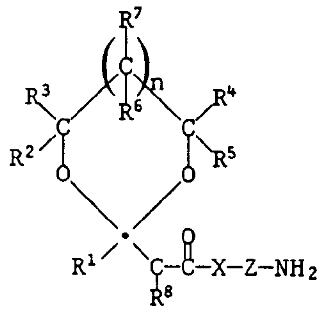

(XII)

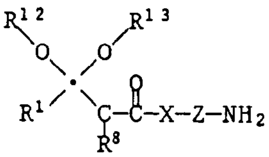

(XIII),

worin n, $R^1$, $R^2$, $R^3$, $R^4$, $R^5$, $R^6$, $R^7$, $R^8$, $R^{12}$, $R^{13}$, X und Z die gleiche Bedeutung wie in Formeln I und II haben, mit Maleinsäureanhydrid, einer Verbindung der Formel IX oder X oder deren Anhydrid in molaren Mengen zu Verbindungen der Formel I oder II umsetzt.

6. Polymere β-Ketoesteracetale mit einem Molekulargewicht (Mw) von 1500 bis 1'000'000, gemessen durch Gelpermeationschromatographie in Polystyrol als Standard, enthaltend, bezogen auf die Gesamtmenge der im Polymer vorhandenen Strukturelemente, 100 bis 5 Mol% des wiederkehrenden Strukturelementes der Formel XIV oder XV

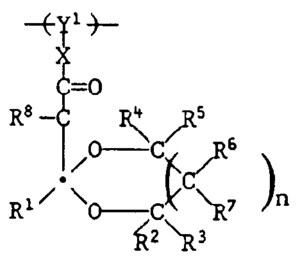

(XIV) oder

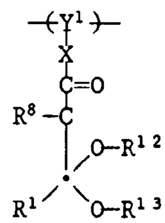

(XV)

und 95 bis 0 Mol% des wiederkehrenden Strukturelementes der Formel XVI

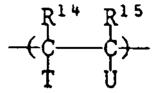

(XVI),

worin n, $R^1$, $R^2$, $R^3$, $R^4$, $R^5$, $R^6$, $R^7$, $R^8$, $R^{12}$, $R^{13}$ und X die gleiche Bedeutung wie in Formel I und II haben und $Y^1$ für einen Rest der folgenden Formeln

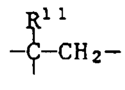 , 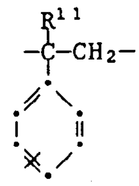 , 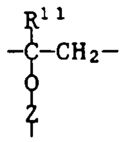 , 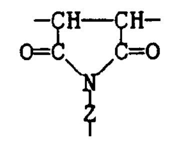 ,

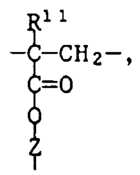 , 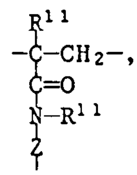 , 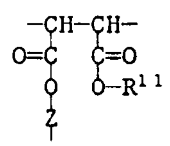 oder 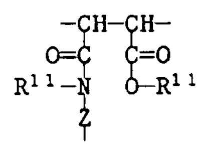 .

steht, worin $R^{11}$ und Z die gleiche Bedeutung wie im Anspruch 1 haben, $R^{14}$ und $R^{15}$ unabhängig voneinander je ein Wasserstoffatom, unsubstituiertes oder durch Halogenatome, Cyano- oder Nitrogruppen substituiertes $C_1$-$C_4$-Alkyl oder ein unsubstituiertes oder durch Halogenatome, $C_1$-$C_4$-Alkoxy, Hydroxy-, Cyano- oder Nitrogruppen substituiertes Phenyl oder Naphthyl bedeuten, T und U unabhängig voneinander je ein Wasserstoffatom, unsubstituierte oder durch Halogenatome, Cyano- oder Nitrogruppen substituiertes $C_1$-$C_{12}$-Alkyl, unsubstituiertes oder durch Halogenatome, Hydroxy-, Cyano-, Nitrogruppen, $C_1$-$C_4$-Alkyl oder $C_1$-$C_4$-Alkoxy substituiertes Phenyl, Naphthyl oder Benzyl oder einen der folgenden Reste $-OR^{18}$, $-COOR^{19}$ oder $-COR^{20}$ bedeuten, wobei $R^{18}$ und $R^{19}$ unabhängig voneinander je ein Wasserstoffatom, unsubstituiertes oder durch Halogenatome, Cyano- oder Nitrogruppen substituiertes $C_1$-$C_{12}$-Alkyl, unsubstituiertes oder durch Halogen, Cyano-, Nitrogruppen, $C_1$-$C_4$-Alkyl oder $C_1$-$C_4$-Alkoxy substituiertes Phenyl oder Naphthyl bedeuten und $R^{20}$ die gleiche Bedeutung wie $R^{18}$ hat und ausserdem für den Rest

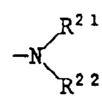

steht, worin $R^{21}$ und $R^{22}$ unabhängig voneinander die gleiche Bedeutung wie $R^{18}$ haben.

7. Strahlungsempfindliches Gemisch enthaltend, bezogen auf die Gesamtmenge der Komponenten a) und b),

a) 85 bis 99 Gew.% eines polymeren β-Ketoesteracetals mit den Strukturelementen der Formel XIV oder XV und gegebenenfalls der Formel XVI gemäss Anspruch 6, und

b) 1 bis 15 Gew.% einer unter Einwirkung von aktinischer Strahlung säurebildenden Substanz.

8. Zusammensetzung gemäss Anspruch 7 enthaltend als zusätzliche Komponente c) ein Bindemittel.

9. Verfahren zur Erzeugung von positiven Abbildungen durch

a) Beschichtung eines Substrates mit einer strahlungsempfindlichen Zusammensetzung gemäss den Ansprüchen 7 oder 8,

b) Belichtung des beschichteten Substrates mit einem vorbestimmten Muster aktinischer Strahlung, und

c) Entwicklung des belichteten Substrates.

10. Verwendung der Zusammensetzung gemäss Ansprüchen 7 oder 8, als Positiv-Resists für die Herstellung von Druckformen, gedruckten Schaltungen oder integrierten Schaltkreisen, sowie für silberlose photographische Filme.

11. Die unter Verwendung der Zusammensetzung gemäss Anspruch 7 hergestellten Druckformen, gedruckten Schaltungen, integrierten Schaltkreise oder silberlosen photographischen Filme.

## Claims

1. A compound of the formula I or II

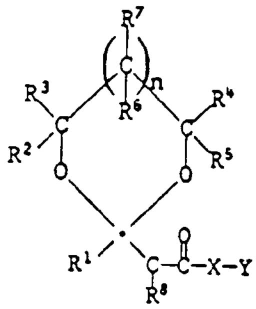

(I)

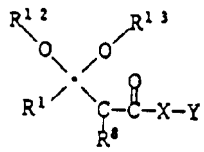

(II)

in which n is zero or the number 1 or 2,
$R^1$ is a hydrogen atom, a $C_1$-$C_{10}$ alkyl which is unsubstituted or substituted by halogen atoms, a $C_2$-$C_{10}$ alkyl which is interrupted by ether oxygen atoms, or a phenyl or benzyl which is unsubstituted or substituted by halogen atoms, nitro groups, $C_1$-$C_4$ alkyl or $C_1$-$C_4$ alkoxy,
$R^2$, $R^3$, $R^4$, $R^5$, $R^6$, $R^7$ and $R^8$ independently of one another are each a hydrogen atom, a halogen atom, a $C_1$-$C_{10}$ alkyl which is unsubstituted or substituted by halogen atoms, a phenyl or naphthyl which is unsubstituted or substituted by halogen atoms, cyano groups, nitro groups or
$C_1$-$C_4$ alkyl, or a radical of the formulae $-COOR^9$,

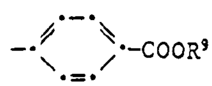

or $-SO_2R^9$, in which
$R^9$ is a $C_1$-$C_6$ alkyl or phenyl which is unsubstituted or substituted by halogen atoms or nitro groups,
X is O, S or $NR^{10}$, $R^{10}$ being a hydrogen atom or a $C_1$-$C_6$ alkyl or phenyl which is unsubstituted or substituted by halogen atoms or nitro groups, and
Y is a radical of the following formulae

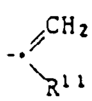 , 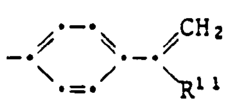 , 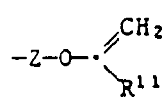 , 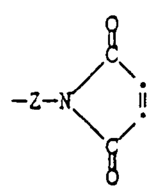 , 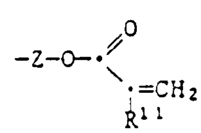 ,

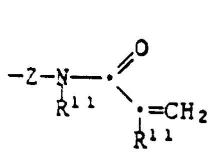 , 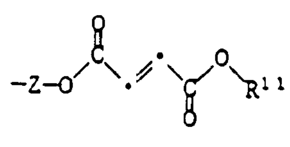 or 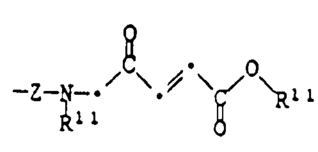

in which $R^{11}$ is a hydrogen atom or a $C_1$-$C_6$ alkyl or phenyl which is unsubstituted or substituted by halogen atoms or nitro groups,
Z is an aliphatic radical which is unsubstituted or substituted by halogen atoms, nitro groups or phenyl and contains at least two methylene groups, or an aliphatic radical which is unsubstituted or substituted by halogen atoms or nitro groups, contains at least two methylene groups and is interrupted by ether oxygen atoms, phenylene or cyclohexylene groups, and
$R^{12}$ and $R^{13}$ independently of one another are each a $C_1$-$C_{10}$ alkyl, phenyl or naphthyl which is unsubstituted or substituted by halogen atoms or nitro groups.

2. A compound of the formula I or II according to claim 1, in which n is zero or the number 1, $R^1$ is a hydrogen atom, a $C_1$-$C_4$ alkyl which is unsubstituted or substituted by a chlorine atom or atoms, a $C_2$-$C_4$ alkyl interrupted by an ether oxygen atom or atoms, phenyl or benzyl, $R^2$, $R^3$, $R^4$, $R^5$, $R^6$ and $R^7$ independently of one another are each a hydrogen atom, chlorine atom, $C_1$-$C_6$ alkyl or phenyl, $R^8$ is a hydrogen atom, phenyl, p-nitrophenyl, p-cyanophenyl or the radical $-COOR^9$, in which $R^9$ is $C_1$-$C_6$ alkyl or phenyl, X is an oxygen atom, Y is a radical of the formulae

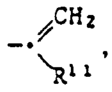 , 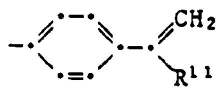 , 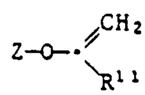

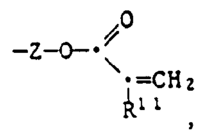 ,

in which $R^{11}$ is a hydrogen atom or $C_1$-$C_4$ alkyl, Z is one of the following radicals

$-(CH_2)_b-$, $-CH_2-CH_2-(OCH_2-CH_2)_c-OCH_2-CH_2-$,

$-CH_2-CHR^{11}-$,

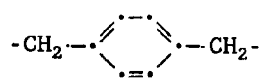 or 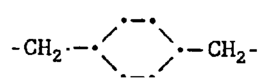 ,

in which b is a number from 3 to 5 and c is zero or a number from 1 to 3, and $R^{12}$ and $R^{13}$ independently of one another are each $C_1$-$C_4$ alkyl.

3. A compound of the formula I according to claim 1, in which n is zero or the number 1, $R^1$ is $C_1$-$C_4$ alkyl, methoxymethyl or phenyl, $R^2$, $R^3$, $R^4$, $R^5$, $R^6$, $R^7$ and $R^8$ are each a hydrogen atom, X is an oxygen atom and Y is the radical

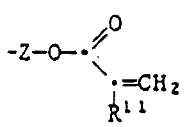

in which Z is ethylene and $R^{11}$ is a hydrogen atom or methyl.

4. A compound of the formula II according to claim 1, in which $R^1$, $R^{12}$ and $R^{13}$ independently of one another are each $C_1$-$C_4$ alkyl, $R^8$ is a hydrogen atom, X is an oxygen atom and Y is a radical of the formula

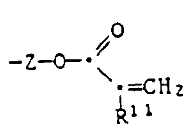

in which Z is ethylene and $R^{11}$ is a hydrogen atom or methyl.

5. A process for preparing a compound of the formula I or II according to claim 1, which comprises

a) reacting a compound of the formula III

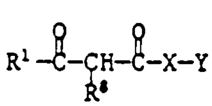 (III)

in which $R^1$, $R^8$, X and Y are as defined in formula I, either with a compound of the formula IV

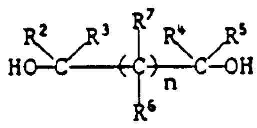

(IV)

in which n, $R^2$, $R^3$, $R^4$, $R^5$, $R^6$ and $R^7$ are as defined in formula I, to give a compound of the formula I, at least 1 mol of the compound of the formula IV being employed per 1 mol of the compound of the formula III, or with compounds of the formulae V and VI

$HO\text{-}R^{12}$ (V)

and

$OH\text{-}R^{13}$ (VI)

in which $R^{12}$ and $R^{13}$ are as defined in formula II, to give a compound of the formula II, at least 2 mol of the compounds of the formulae V and VI being employed per 1 mol of the compound III,
b) reacting a compound of the formula VII or VIII

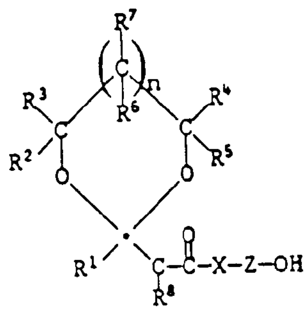

(VII)

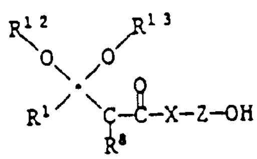

(VIII)

in which n, $R^1$, $R^2$, $R^3$, $R^4$, $R^5$, $R^6$, $R^7$, $R^8$, $R^{12}$, $R^{13}$, X and Z are as defined in formulae I or II, with a compound of the formula IX, X or XI

(IX)

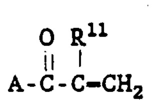

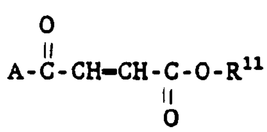

(X) or

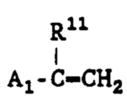

(XI)

in which $R^{11}$ is as defined in formula I or II, A is a halogen atom or a hydroxyl group, it also being possible for the compounds of the formulae IX or X to be in the form of an anhydride, and $A_1$ is a halogen atom or $C_1$-$C_4$ alkoxy, in molar quantities to give a compound of the formula I or II, or
c) reacting a compound of the formula XII or XIII

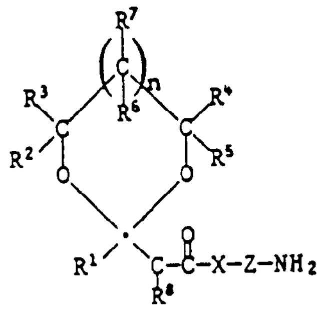

(XII)

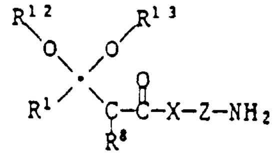

(XIII)

in which n, $R^1$, $R^2$, $R^3$, $R^4$, $R^5$, $R^6$, $R^7$, $R^8$, $R^{12}$, $R^{13}$, X and Z are as defined in formulae I and II, with maleic anhydride, a compound of the formula IX or X or the anhydride thereof in molar quantities to give a compound of the formula I or II.

6. A polymeric β-keto-ester acetal having a molecular weight (Mw) from 1500 to 1,000,000, measured by gel permeation chromatography in polystyrene as standard, containing, relative to the total quantity of the structural units present in the polymer, 100 to 5 mol% of the recurrent structural unit of the formula XIV or XV

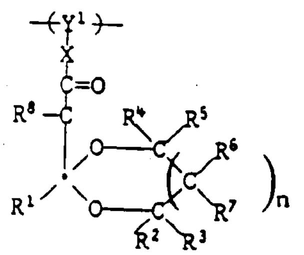

(XIV)

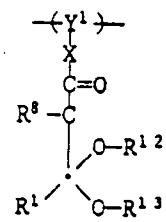

(XV)

and 95 to 0 mol% of the recurrent structural unit of the formula XVI

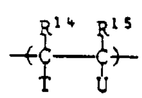

(XVI)

in which n, $R^1$, $R^2$, $R^3$, $R^4$, $R^5$, $R^6$, $R^7$, $R^8$, $R^{12}$, $R^{13}$ and X are as defined in formulae I and II, and $Y^1$ is a radical of the following formulae

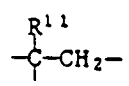 , 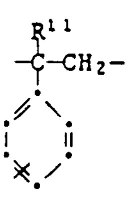 , 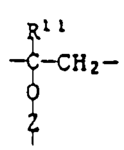 , 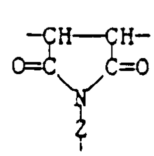 ,

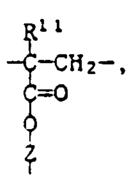 , 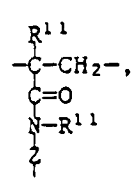 , 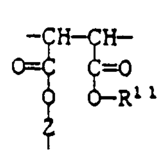 or 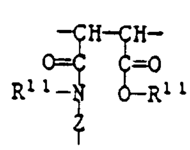

in which $R^{11}$ and Z are as defined in claim 1, $R^{14}$ and $R^{15}$ independently of one another are each a hydrogen

atom, a $C_1$-$C_4$ alkyl which is unsubstituted or substituted by halogen atoms, cyano groups or nitro groups, or a phenyl or naphthyl which is unsubstituted or substituted by halogen atoms, $C_1$-$C_4$ alkoxy, hydroxyl groups, cyano groups or nitro groups, T and U independently of one another are each a hydrogen atom, a $C_1$-$C_{12}$ alkyl which is unsubstituted or substituted by halogen atoms, hydroxyl groups, cyano groups or nitro groups, a phenyl, naphthyl or benzyl which is unsubstituted or substituted by halogen atoms, cyano groups, nitro groups, $C_1$-$C_4$ alkyl or $C_1$-$C_4$ alkoxy, or one of the following radicals $-OR^{18}$, $-COOR^{19}$ or $-COR^{20}$, in which $R^{18}$ and $R^{19}$ independently of one another are each a hydrogen atom, a $C_1$-$C_{12}$ alkyl which is unsubstituted or substituted by halogen atoms, cyano groups or nitro groups or a phenyl or naphthyl which is unsubstituted or substituted by halogen, cyano groups, nitro groups, $C_1$-$C_4$ alkyl or $C_1$-$C_4$ alkoxy, and $R^{20}$ is as defined for $R^{18}$ or is a radical

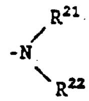 ,

in which $R^{21}$ and $R^{22}$ independently of one another are as defined for $R^{18}$.

7. A radiation-sensitive mixture containing, relative to the total quantity of components a) and b),

a) 85 to 99% by weight of a polymeric β-keto-ester acetal with the structural units of the formula XIV or XV and, if appropriate, of the formula XVI according to claim 6, and

b) 1 to 15% by weight of a substance which forms acid under the action of actinic radiation.

8. A composition according to claim 7, containing a binder as an additional component c).

9. A process for producing positive images by

a) coating a substrate with a radiation-sensitive composition according to claim 7 or 8,

b) exposure of the coated substrate with a predetermined pattern of actinic radiation and

c) developing the coated substrate.

10. The use of a composition according to either of claims 7 and 8 as a positive resist for the production of printing formes, printed circuit boards, integrated circuits or silver-free photographic films.

11. The printing formes, printed circuit boards, integrated circuits and silver-free photographic films produced with the use of the composition according to claim 7.

**Revendications**

1. Composés répondant à l'une des formules I et II :

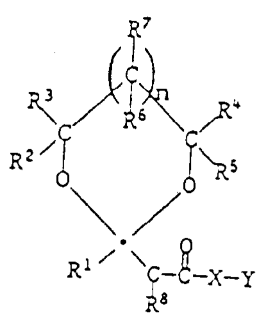

(I)

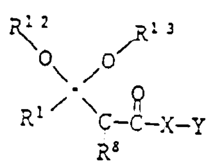

(I)

dans lesquelles

n désigne un nombre égal à 0, à 1 ou à 2,

$R^1$ représente un atome d'hydrogène, un alkyle en $C_1$-$C_{10}$ non substitué ou porteur d'atomes d'halogènes, un alkyle en $C_2$-$C_{10}$ interrompu par des atomes d'oxygène de fonction éther, ou un radical phényle ou benzyle non substitué ou porteur d'atomes d'halogènes, de radicaux nitro, d'alkyles en $C_1$-$C_4$ ou d'alcoxy en $C_1$-$C_4$,

$R^2$, $R^3$, $R^4$, $R^5$, $R^6$, $R^7$ et $R^8$ représentent chacun, indépendamment les uns des autres, un atome d'hydrogène, un atome d'halogène, un alkyle en $C_1$-$C_{10}$ non substitué ou porteur d'atomes d'halogènes, un radical phényle ou naphtyle non substitué ou porteur d'atomes d'halogènes, de radicaux cyano, de radicaux nitro ou d'alkyles en $C_1$-$C_4$, ou un radical répondant à l'une des formules -$COOR^9$

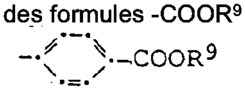

ou -$SO_2R^9$ dans lesquelles $R^9$ représente un alkyle en $C_1$-$C_6$ ou un phényle, l'un et l'autre pouvant être dépourvus de substituant ou porter des atomes d'halogènes ou des radicaux nitro,
X représente O, S ou $NR^{10}$, le symbole $R^{10}$ désignant un atome d'hydrogène, un alkyle en $C_1$-$C_6$ ou un phényle, chacun de ces deux derniers pouvant être dépourvu de substituant ou porter des atomes d'halogènes ou des radicaux nitro,
Y représente un radical répondant à l'une des formules suivantes :

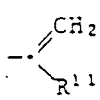 , 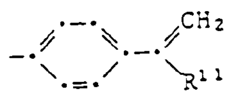 , 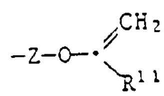 , 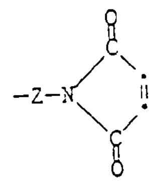 , 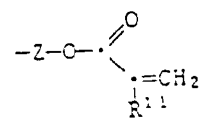 ,

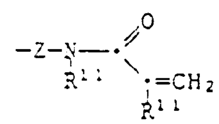 , 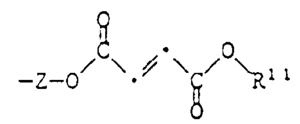 et 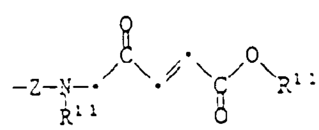

dans lesquelles $R^{11}$ représente un atome d'hydrogène ou représente un alkyle en $C_1$-$C_6$ ou un phényle qui peuvent chacun être dépourvus de substituant ou porter des atomes d'halogènes ou des radicaux nitro, et Z représente un radical aliphatique contenant au moins deux radicaux méthylènes, non substitué ou porteur d'atomes d'halogènes, de radicaux nitro ou d'un phényle, ou un radical aliphatique interrompu par des atomes d'oxygène de fonction éther ou par des radicaux phénylènes ou cyclohexylènes, non substitué ou porteur d'atomes d'halogènes ou de radicaux nitro, et
$R^{12}$ et $R^{13}$ représentent chacun, indépendamment l'un de l'autre, un alkyle en $C_1$-$C_{10}$, un phényle ou un naphtyle, qui peuvent chacun être dépourvus de substituant ou porter des atomes d'halogènes ou des radicaux nitro.

2. Composés de formule I ou II selon la revendication I, dans lesquels n est égal à 0 ou à 1, $R^1$ représente un atome d'hydrogène, un alkyle en $C_1$-$C_4$ non substitué ou porteur d'un ou de plusieurs atomes de chlore, un alkyle en $C_2$-$C_4$ interrompu par un ou plusieurs atomes d'oxygène de fonction éther, un phényle ou un benzyle, $R^2$, $R^3$, $R^4$, $R^5$, $R^6$ et $R^7$ représentent chacun, indépendamment les uns des autres, un atome d'hydrogène, un atome de chlore, un alkyle en $C_1$-$C_6$ ou un phényle, $R^8$ représente un atome d'hydrogène, un phényle, un nitro-4 phényle, un cyano-4 phényle ou un radical -$COOR^9$ dans lequel $R^9$ représente un alkyle en $C_1$-$C_6$ ou un phényle, X représente un atome d'oxygène, Y représente un radical répondant à l'une des formules :

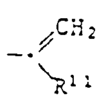 , 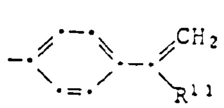 , 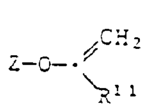 et 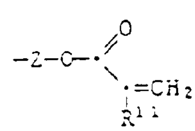

dans lesquelles $R^{11}$ représente un atome d'hydrogène ou un alkyle en $C_1$-$C_4$, Z représente un des radicaux suivants :

$-CH_2-CH_2-(OCH_2-CH_2)_c-OCH_2-CH_2-$ , $-CH_2-CHR^{11}-$ , 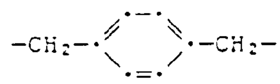

et 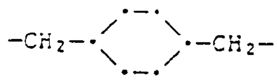

dans lesquels b désigne un nombre de 3 à 5 et c un nombre de 0 à 3, et $R^{12}$ et $R^{13}$ représentent chacun, indépendamment l'un de l'autre, un alkyle en $C_1$-$C_4$.

3. Composés de formule I selon la revendication 1 dans lesquels n est égal à 0 ou à 1, $R^1$ représente un alkyle en $C_1$-$C_4$, un méthoxyméthyle ou un phényle, $R^2$, $R^3$, $R^4$, $R^5$, $R^6$, $R^7$ et $R^8$ représentent chacun un atome d'hydrogène, X représente un atome d'oxygène et Y représente un radical

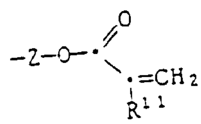

dans lequel Z représente un radical éthylène et $R^{11}$ un atome d'hydrogène ou un méthyle.

4. Composés de formule II selon la revendication 1 dans lesquels $R^1$, $R^{12}$ et $R^{13}$ représentent chacun, indépendamment l'un de l'autre, un alkyle en $C_1$-$C_4$, $R^8$ représente un atome d'hydrogène, X représente un atome d'oxygène et Y représente un radical

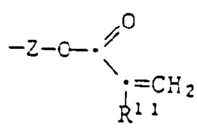

dans lequel Z représente l'éthylène et $R^{11}$ un atome d'hydrogène ou un méthyle.

5. Procédé pour préparer des composés de formule I ou II selon la revendication 1, procédé caractérisé en ce que :

a) on fait réagir un composé répondant à la formule III :

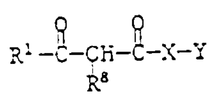 (III)

dans laquelle $R^1$, $R^8$, X et Y ont les mêmes significations que dans la formule I, soit avec un composé de formule IV :

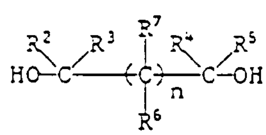 (IV)

dans lequel n, $R^2$, $R^3$, $R^4$, $R^5$, $R^6$ et $R^7$ ont les mêmes significations que dans la formule I, pour obtenir un composé de formule I, en mettant en jeu, pour 1 mol du composé de formule III, au moins 1 mol d'un composé de formule IV, soit avec des composés de formules V et VI :

$HO-R^{12}$ (V)

$HO-R^{13}$ (VI)

dans lesquelles $R^{12}$ et $R^{13}$ ont les mêmes significations que dans la formule II, réaction qui conduit à un composé de formule II, en mettant en jeu, pour 1 mol du composé de formule III, au moins 2 mol des composés de formules V et VI,

b) on fait réagir des composés répondant à l'une des formules VII et VIII :

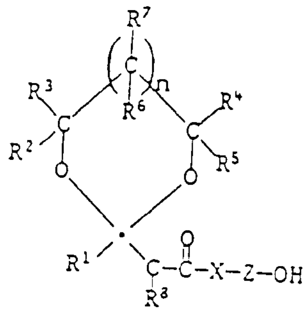

(VII)

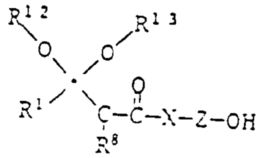

(VIII)

dans lesquelles n, $R^1$, $R^2$, $R^3$, $R^4$, $R^5$, $R^6$, $R^7$, $R^8$, $R^{12}$, $R^{13}$, X et Z ont les mêmes significations que dans les formules I et II, avec un composé répondant à l'une des formules IX, X et XI :

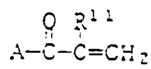

(IX)

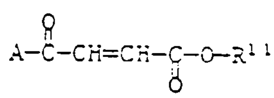

(X)

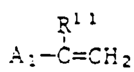

(XI)

dans lesquelles $R^{11}$ a la même signification que dans la formule I ou II, A représente un atome d'halogène ou un radical hydroxy, les composés de formules IX et X pouvant également être sous la forme d'un anhydride, et $A_1$ représente un atome d'halogène ou un alcoxy en $C_1$-$C_4$, en des quantités molaires, de manière à obtenir des composés de formule I ou II, ou

c) on fait réagir des composés répondant à l'une des formules XII et XIII :

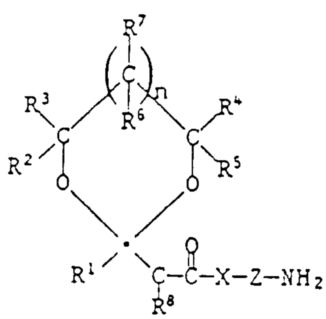

(XII)

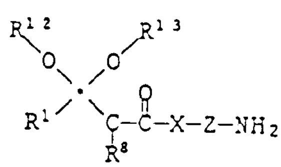

(XIII)

dans lesquelles n, $R^1$, $R^2$, $R^3$, $R^4$, $R^5$, $R^6$, $R^7$, $R^8$, $R^{12}$, $R^{13}$, X et Z ont les mêmes significations que dans les formules I et II, avec l'anhydride maléique, un composé de formule IX ou X ou son anhydride, en des quantités molaires, de manière à obtenir des composés de formule I ou II.

6. β-Céto-ester-acétals polymères ayant une masse moléculaire (Mp) de 1500 à 1 000 000, mesurée par chromatographie d'exclusion (perméation sur gel) dans du polystyrène comme référence, qui contiennent, par rapport à la quantité totale des unités structurales présentes dans le polymère, de 100 à 5 % en moles de l'unité structurale répétée répondant à la formule XIV ou XV :

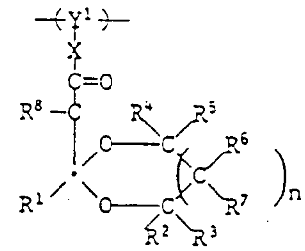 (XIV) 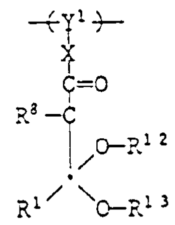 (XV)

et de 95 à 0 % en moles de l'unité structurale répétée de formule XVI :

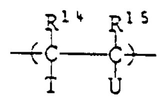 (XVI),

formules dans lesquelles n, $R^1$, $R^2$, $R^3$, $R^4$, $R^5$, $R^6$, $R^7$, $R^8$, $R^{12}$, $R^{13}$ et X ont les mêmes significations que dans les formules I et II, $Y^1$ représente un radical répondant à l'une des formules :

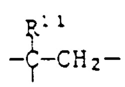, 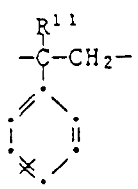, 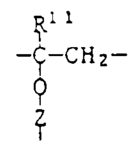, 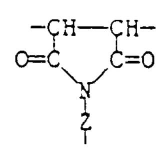,

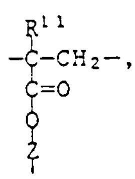, 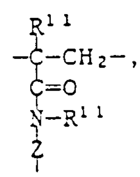, 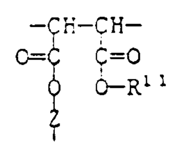 et 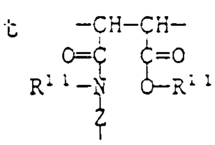

(dans lesquelles $R^{11}$ et Z ont les significations qui leur ont été données dans la revendication 1), $R^{14}$ et $R^{15}$ représentent chacun, indépendamment l'un de l'autre, un atome d'hydrogène, un alkyle en $C_1$-$C_4$ non substitué ou porteur d'atomes d'halogènes ou de radicaux cyano ou nitro, ou un radical phényle ou naphtyle non substitué ou porteur d'atomes d'halogènes ou de radicaux alcoxy en $C_1$-$C_4$, hydroxy, cyano ou nitro, T et U représentent chacun, indépendamment l'un de l'autre, un atome d'hydrogène, un alkyle en $C_1$-$C_{12}$ non substitué ou porteur d'atomes d'halogènes ou de radicaux cyano ou nitro, un radical phényle, naphtyle ou benzyle non substitué ou porteur d'atomes d'halogènes ou de radicaux hydroxy, cyano, nitro, alkyles en $C_1$-$C_4$ ou alcoxy en $C_1$-$C_4$, ou un des radicaux -$OR^{18}$, -$COOR^{19}$ et -$COR^{20}$ dans lesquels $R^{18}$ et $R^{19}$ représentent chacun, indépendamment l'un de l'autre, un atome d'hydrogène, un alkyle en $C_1$-$C_{12}$ non substitué ou porteur d'atomes d'halogènes ou de radicaux cyano ou nitro, un radical phényle ou naphtyle non substitué ou porteur d'atomes d'halogènes ou de radicaux cyano, nitro, alkyles en $C_1$-$C_4$ ou alcoxy en $C_1$-$C_4$, et $R^{20}$ a la même signification que $R^{18}$ et représente en outre un radical

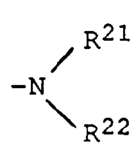

dans lequel $R^{21}$ et $R^{22}$ ont chacun, indépendamment l'un de l'autre, la même signification que $R^{18}$.

7. Mélange photosensible qui contient, par rapport à la quantité totale des composantes a) et b) :

a) de 85 à 99 % en poids d'un β-céto-ester-acétal polymère contenant les unités structurales de formule XIV ou XV et, éventuellement, de formule XVI selon la revendication 6, et

b) de 1 à 15 % en poids d'une substance capable de former un acide sous l'action d'un rayonnement actinique.

8. Composition selon la revendication 7 qui contient un liant comme composante supplémentaire c).

9. Procédé pour créer des images positives, procédé selon lequel :

a) on revêt un substrat avec une composition photosensible selon l'une des revendications 7 et 8,

b) on expose le substrat revêtu à un rayonnement actinique selon un modèle déterminé, et

c) on développe le substrat irradié.

10. Application de la composition selon l'une des revendications 7 et 8 comme résist positif pour la fabrication de formes d'impression, de circuits imprimés et de circuits intégrés ainsi que de pellicules photographiques non argentiques.

11. Formes d'impression, circuits imprimés, circuits intégrés et pellicules photographiques non argentiques qui ont été fabriqués à l'aide de la composition selon la revendication 7.